# EUROPEAN PATENT SPECIFICATION

(11) **EP 4 173 607 B1**
(45) Date of publication and mention of the grant of the patent: **01.07.2026**
(21) Application number: 21875816.7
(22) Date of filing: 30.09.2021
(51) Int. Cl.: A61F 13/49, A61F 13/51

(54) **ABSORBENT ARTICLE**
ABSORBIERENDER ARTIKEL
ARTICLE ABSORBANT

(30) Priority: 30.09.2020 JP 2020165869
(43) Date of publication of application: 03.05.2023
(73) Proprietor: Unicharm Corporation, Ehime 799-0111 (JP)
(72) Inventor: TANAKA, Suguru, Kanonji-shi, Kagawa 769-1602 (JP); MITSUNO, Satoshi, Kanonji-shi, Kagawa 769-1602 (JP)
(74) Representative: Dolleymores
(86) International application number: PCT/JP2021/036274
(87) International publication number: WO 2022/071520

(56) References cited:
- WO-A1-2018/116479
- WO-A1-2018/235211
- WO-A1-2019/169989
- JP-A- 2004 249 079
- JP-A- 2015 066 008

## Description

### FIELD

The present invention relates to an absorbent article.

### BACKGROUND

There has been conventionally known an underpants-shaped diaper using a hydrophilic nonwoven fabric having enhanced hydrophilicity. For example, Patent Literature 1 discloses a technique related to a disposable diaper in which a hydrophilic nonwoven fabric is used as an exterior sheet 4 that constitutes an exterior body 3 of a diaper 1, the hydrophilic nonwoven fabric having hydrophilized fibers which are obtained by means such as making hydrophobic synthetic fibers undergo treatment with a hydrophilizing agent.

### [CITATION LIST]

### [PATENT LITERATURE]

Patent Literature 1: Japanese Patent Application Publication No. 2017-113186
Patent Literature 2: WO2019169989

### SUMMARY

### [TECHNICAL PROBLEM]

In such a diaper using a hydrophilic sheet member, moisture such as sweat from the wearer's skin can be absorbed while the diaper is put on. However, even when moisture is absorbed from the wearer's skin by the hydrophilic sheet, there is a problem that moisture retained in the hydrophilic sheet is less likely to be evaporated. In this case, there is a risk that the hydrophilic sheet containing moisture comes into contact with the wearer's skin to make the wearer feel discomfort.

The present invention was achieved in light of conventional problems such as that described above, and an aspect of the present invention is to efficiently evaporate moisture in an absorbent article including a hydrophilic nonwoven fabric.

### [SOLUTION TO PROBLEM]

A main aspect of the present invention for achieving the above-described aspect is an absorbent article including: a liquid-absorbent absorbent main body; and a waist member that is provided on a non-skin side with respect to the absorbent main body, the waist member having a hydrophobic nonwoven fabric and a hydrophilic nonwoven fabric at least in a partial region, the hydrophilic nonwoven fabric that is overlaid to be adjacent to a non-skin side of the hydrophobic nonwoven fabric and that has higher hydrophilicity than the hydrophobic nonwoven fabric, the waist member having a portion where the hydrophilic nonwoven fabric and the hydrophobic nonwoven fabric are made adhere to each other with an adhesive, a maximum adhesive width by the adhesive in the hydrophilic nonwoven fabric being narrower than a maximum adhesive width by the adhesive in the hydrophobic nonwoven fabric. Features of the present invention other than the above will become clear by reading the description of the present specification with reference to the accompanying drawings. The absorbent article has a vertical direction and a lateral direction that intersect with each other, the waist member has a waist elastic member that stretches and contracts in the lateral direction, the adhesive is provided along the waist elastic member, and a width of a cross section of the waist elastic member that is in a state of being stretched in the lateral direction is narrower than the maximum adhesive width by the adhesive on the hydrophobic nonwoven fabric. The waist member has a second skin-side region that is a region between an outer end of the adhesive provided in the hydrophobic nonwoven fabric and an outer end of the waist elastic member, a second non-skin-side region that is a region facing the second skin-side region in the hydrophilic nonwoven fabric, and a first non-skin-side region that is a region having a predetermined width and adjacent to an outside of the second non-skin-side region in the hydrophilic nonwoven fabric, and an average density of the hydrophilic nonwoven fabric in the second non-skin-side region is greater than an average density of the hydrophilic nonwoven fabric in the first non-skin-side region.

### [ADVANTAGEOUS EFFECT OF THE INVENTION]

According to the present invention, in an absorbent article including a hydrophilic nonwoven fabric, it is possible to efficiently evaporate moisture.

### BRIEF DESCRIPTION OF DRAWINGS

FIG. 1 is a schematic perspective view of a diaper 1.
FIG. 2 is a plan view of the diaper 1 in an unfolded and stretched state.
FIG. 3 is a schematic cross-sectional view taken along a line A-A in FIG. 2.
FIG. 4A is a plan view of an absorbent main body 10, and FIG. 4B is a schematic cross-sectional view of the absorbent main body 10.
FIGS. 5A to 5C are diagrams illustrating the basic principle when moisture is absorbed and evaporated in the diaper 1.
FIG. 6 is a schematic cross-sectional view showing a vicinity of a joining portion between a skin-side sheet 31 (hydrophobic nonwoven fabric) and a non-skin-side sheet 32 (hydrophilic nonwoven fabric) in an enlarged manner.
FIG. 7 is data of measurement results on a transverse cross section (CD cross section) of a waist member 20.
FIGS. 8A and 8B are diagrams illustrating the configuration of a buttocks cover 40b of a back waist portion 40.
FIG. 9A is a plan view of a diaper 2 in an unfolded and stretched state.
FIG. 9B is a schematic cross-sectional view taken along a line D-D in FIG. 9A.
FIG. 10A is a plan view of a diaper 3 in an unfolded and stretched state.
FIG. 10B is a schematic cross-sectional view taken along a line E-E in FIG. 10A.

### DESCRIPTION OF EMBODIMENTS

At least following matters will become clear with description of this specification and attached drawings.

An absorbent article including: a liquid-absorbent absorbent main body; and a waist member that is provided on a non-skin side with respect to the absorbent main body, the waist member having a hydrophobic nonwoven fabric and a hydrophilic nonwoven fabric at least in a partial region, the hydrophilic nonwoven fabric that is overlaid to be adjacent to a non-skin side of the hydrophobic nonwoven fabric and that has higher hydrophilicity than the hydrophobic nonwoven fabric, the waist member having a portion where the hydrophilic nonwoven fabric and the hydrophobic nonwoven fabric are made adhere to each other with an adhesive, a maximum adhesive width by the adhesive in the hydrophilic nonwoven fabric being narrower than a maximum adhesive width by the adhesive in the hydrophobic nonwoven fabric.

According to the above-described absorbent article, the hydrophilic nonwoven fabric and the hydrophobic nonwoven fabric are joined with the adhesive and are in close contact with each other, and this makes moisture such as sweat absorbed by the hydrophobic nonwoven fabric located on the skin side more likely to be smoothly transferred to the hydrophilic nonwoven fabric located on the non-skin side. Then, the transferred moisture is diffused in the hydrophilic nonwoven fabric, and evaporated from the non-skin-side surface of the hydrophilic nonwoven fabric to the outside (atmospheric side). At this time, the adhesive has a narrow adhesive width in the hydrophilic nonwoven fabric, and this makes it less likely to hinder the movement of moisture compared with the case where the adhesive width is wide, making it more likely to widely diffuse moisture in the hydrophilic nonwoven fabric. Accordingly, in the waist member of the absorbent article, moisture can be efficiently evaporated.

The absorbent article has a vertical direction and a lateral direction that intersect with each other, that the waist member has a waist elastic member that stretches and contracts in the lateral direction, that the adhesive is provided along the waist elastic member, and that a width of a cross section of the waist elastic member that is in a state of being stretched in the lateral direction is narrower than the maximum adhesive width by the adhesive on the hydrophobic nonwoven fabric.

According to the above-described absorbent article, since the area of a portion of the hydrophobic nonwoven fabric where adhesive is applied is large, the density of the hydrophobic nonwoven fabric on the skin surface side is increased and the contractive force of the elastic member is hindered, increasing the smoothness. Therefore, the contact area between the hydrophobic nonwoven fabric and the skin is increased, and liquid is likely to be absorbed. Accordingly, the effect of absorbing and evaporating moisture can be further enhanced.

The waist member has a second skin-side region that is a region between an outer end of the adhesive provided in the hydrophobic nonwoven fabric and an outer end of the waist elastic member, a second non-skin-side region that is a region facing the second skin-side region in the hydrophilic nonwoven fabric, and a first non-skin-side region that is a region having a predetermined width and adjacent to an outside of the second non-skin-side region in the hydrophilic nonwoven fabric, and that an average density of the hydrophilic nonwoven fabric in the second non-skin-side region is greater than an average density of the hydrophilic nonwoven fabric in the first non-skin-side region.

According to the above-described absorbent article, in the hydrophilic nonwoven fabric, the average density of the second non-skin-side region is greater than the average density of the first non-skin-side region, and this makes moisture more likely to move (be diffused) from the first non-skin-side region side to the second non-skin-side region side. Further, the moisture is more likely to move (be diffused) in the second non-skin-side region along the waist elastic member. This makes it more likely to widely diffuse moisture in the hydrophilic nonwoven fabric, making it possible to further increase the evaporation efficiency of moisture in the hydrophilic nonwoven fabric.

In such an absorbent article, it is desirable that at least a part of the hydrophilic nonwoven fabric is arranged farthest on a non-skin side of the waist member.

According to the above-described absorbent article, since the absorbent article has a portion in which no other member is interposed between the hydrophilic nonwoven fabric and the atmosphere, this portion serves as an interface with the atmosphere, making it more likely to evaporate moisture which is retained in the hydrophilic nonwoven fabric, into the atmosphere. Therefore, the moisture can be efficiently evaporated into the atmosphere.

In such an absorbent article, it is desirable that the waist member has a front waist portion and a back waist portion, and that a farthest-on-non-skin-side layer of each of the front waist portion and the back waist portion is formed of one sheet of hydrophilic nonwoven fabric.

According to the above-described absorbent article, not providing extra sheet member in the waist member makes it less likely to inhibit the absorption and evaporation of moisture. Further, since the hydrophilic region provided in the non-skin-side surface of the waist member is formed of one sheet of hydrophilic nonwoven fabric, the hydrophilic region is formed in a wide range of the waist member, making it possible to further enhance evaporation property.

In such an absorbent article, it is desirable that the absorbent article has a vertical direction and a lateral direction that intersect with each other, that the absorbent main body has an absorbent core, that the waist member has a front waist portion and a back waist portion, that the front waist portion and the back waist portion are annularly joined by a pair of side joining portions that are provided in two end portions in the lateral direction, and that concerning an upper waist region that overlaps the side joining portion of the waist member with respect to the vertical direction and that is located above an upper end of the absorbent core in the vertical direction, concerning a lower waist region that overlaps the side joining portion of the waist member with respect to the vertical direction and that is located below the upper end of the absorbent core in the vertical direction, a ratio of a total area of the adhesive arranged in the upper waist region in a linear or dotted manner to an area of the upper waist region is larger than a ratio of a total area of the adhesive arranged in the lower waist region in a linear or dotted manner to an area of the lower waist region.

According to the above-described absorbent article, in the upper waist region, the hydrophobic nonwoven fabric and the hydrophilic nonwoven fabric are more likely to be in close contact with each other in the thickness direction compared with in the lower waist region. Therefore, the function of absorbing moisture from the wearer's skin and evaporating the moisture during usage of the absorbent article is further enhanced in the upper waist region than in the lower waist region. Accordingly, in the wearer's body, in the vicinity of the waist where a large amount of sweat is produced, moisture can be more likely to be efficiently absorbed and evaporated.

In such an absorbent article, it is desirable that the waist member has a plurality of waist elastic members that stretch and contract in the lateral direction, and that a vertical pitch between the plurality of waist elastic members that are provided in the upper waist region is narrower than a vertical pitch between the plurality of waist elastic members that are provided in the lower waist region.

According to the above-described absorbent article, the contractive force acting on the upper waist region by the waist elastic member is likely to be larger than the contractive force acting on the lower waist region. Therefore, the amount of contraction of the hydrophobic nonwoven fabric in the upper waist region becomes larger than the amount of contraction of the hydrophobic nonwoven fabric in the lower waist region, making it more likely to promote the absorption of moisture (sweat) due to a capillary phenomenon. Accordingly, in the vicinity of the waist where a large amount of sweat is produced, moisture is more likely to be absorbed, and this can make it stuffiness or rashes less likely to occur while the absorbent article is put on.

In such an absorbent article, it is desirable that the back waist portion includes a buttocks cover below lower ends of the side joining portions, the buttocks cover having a lateral width that is narrowed from an upper side to a lower side in the vertical direction, the back waist portion includes a back leg elastic member and a waist elastic member, the back leg elastic member stretching and contracting along an end edge portion of the buttocks cover, the waist elastic member stretching and contracting along the lateral direction, and that the back leg elastic member has a portion that intersects the waist elastic member, in the side joining portion.

According to the above-described absorbent article, in the side joining portions where a large number of materials (sheet members) are overlaid on each other and where stuffiness is more likely to occur, the portion in which the waist elastic member and the back leg elastic member intersect is provided. This makes it possible to exert the contractive force of the two types of elastic member on the side joining portion. Accordingly, the contractive force in the side joining portion is increased, and the hydrophobic nonwoven fabric of the back waist portion contracts, decreasing the inter-fiber distance. This can make more likely to absorb moisture such as sweat from the wearer's body.

In such an absorbent article, it is desirable that in an outer edge portion of the buttocks cover, the absorbent article has a portion in which the hydrophilic nonwoven fabric and the hydrophobic nonwoven fabric are compressed.

According to the above-described absorbent article, the hydrophobic nonwoven fabric and the hydrophilic nonwoven fabric are in close contact with each other in the outer edge portion of the buttocks cover, and therefore the transfer of moisture from the hydrophobic nonwoven fabric to the hydrophilic nonwoven fabric is likely to occur. Accordingly, in the buttocks cover, the function of absorbing and evaporating moisture such as sweat from the wearer's body can be enhanced.

In such an absorbent article, it is desirable that in an outer edge portion of the buttocks cover, the absorbent article has a portion where the hydrophilic nonwoven fabric and the hydrophobic nonwoven fabric are joined by an adhesive and a portion where the hydrophilic nonwoven fabric and the hydrophobic nonwoven fabric are not joined.

According to the above-described absorbent article, in a region where the hydrophilic nonwoven fabric and the hydrophobic nonwoven fabric are joined in the outer edge portion of the buttocks cover, the buttocks cover has a sufficient strength, and the hydrophilic nonwoven fabric and the hydrophobic nonwoven fabric are less likely to be peeled off even when the wearer moves the body. On the other hand, in a region where the hydrophilic nonwoven fabric and the hydrophobic nonwoven fabric are not joined, the buttocks cover has flexibility. Therefore, the buttocks cover is likely to fit to the wearer's body, and the wearer can feel a soft texture.

In such an absorbent article, it is desirable that the absorbent article has a vertical direction and a lateral direction that intersect with each other, and that a skin surface sheet formed of a hydrophobic nonwoven fabric is provided on a skin side of the waist member, so as to straddle an upper end of the absorbent main body in the vertical direction.

According to the above-described absorbent article, since the skin surface sheet (hydrophobic nonwoven fabric) is arranged so as to straddle the upper end of the absorbent main body, a top sheet containing moisture (sweat) is suppressed from coming into contact with the wearer's skin in the upper end portion of the absorbent main body. Accordingly, this can make the wearer less likely to feel discomfort while the absorbent article is put on.

In such an absorbent article, it is desirable that the absorbent article has a portion in which the skin surface sheet and the adhesive overlap when viewed in a thickness direction of the waist member.

According to the above-described absorbent article, even in the case where moisture is diffused along the waist elastic member to which the adhesive is applied, in a portion where the skin surface sheet is provided on the non-skin side of the waist elastic member, the transfer of the diffused moisture to the skin side of the wearer (rewetting) is suppressed, and this can make the wearer less likely to feel discomfort.

In such an absorbent article, it is desirable that the skin surface sheet is provided spaced apart from an upper end of the waist member by a predetermined distance in the vertical direction.

According to the above-described absorbent article, since the skin surface sheet is provided spaced apart from the upper end of the waist member, the transfer of moisture from the skin side to the non-skin side is prevented from being hindered by the skin surface sheet in the upper end portion of the waist member. Therefore, moisture (sweat) in the vicinity of the waist where a large amount of sweat is produced can be efficiently absorbed and evaporated toward the non-skin side.

### First Embodiment

The following describes an absorbent article according to the present invention by way of example of a disposable diaper (hereinafter also referred to as a "diaper 1"). However, the absorbent article according to the present invention includes napkins, panty liners, and other types of absorbent article.

### Configuration of Diaper 1

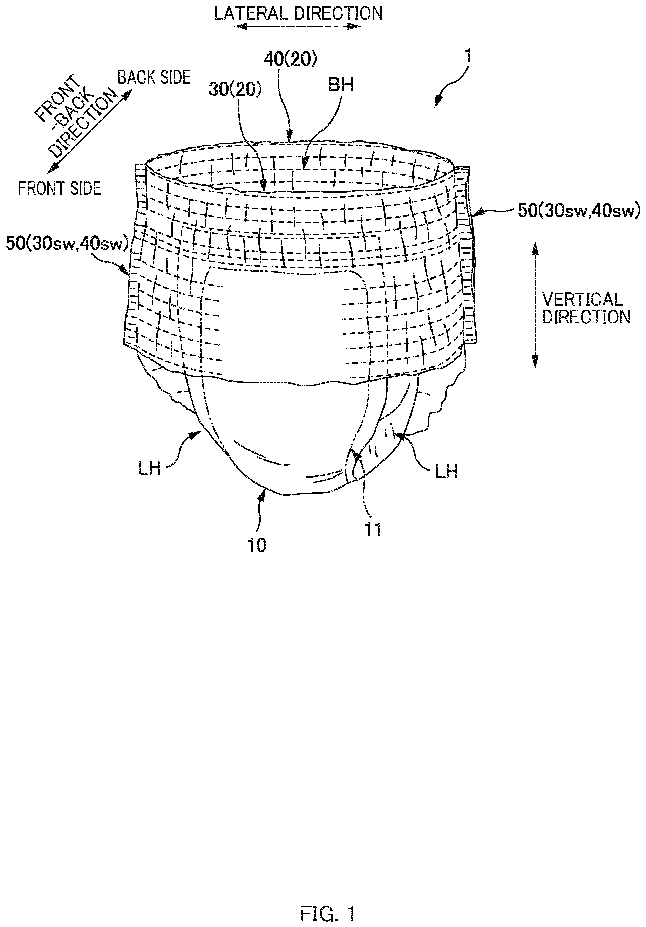

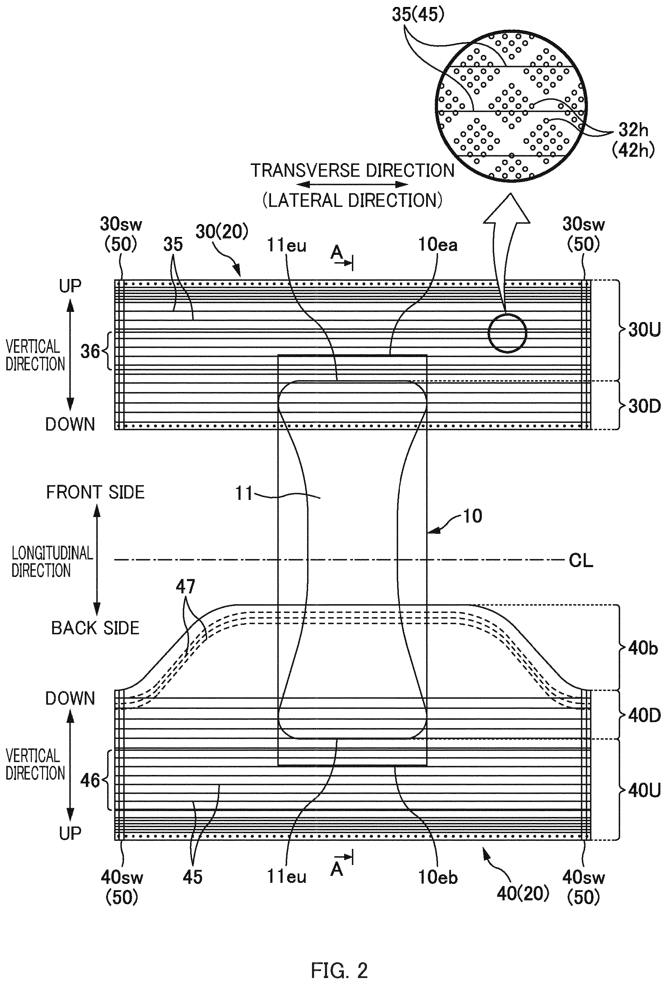

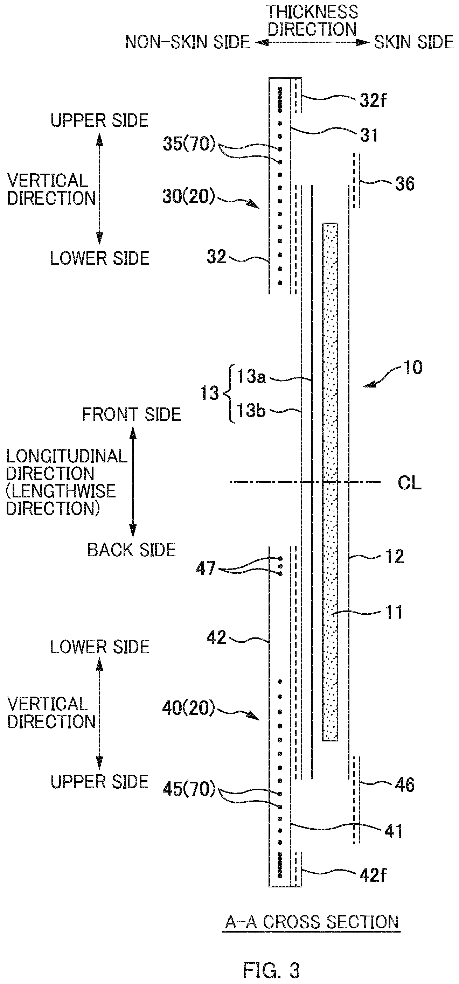

FIG. 1 is a schematic perspective view of the diaper 1. FIG. 2 is a plan view of the diaper 1 in an unfolded and stretched state. FIG. 3 is a schematic cross-sectional view taken along a line A-A in FIG. 2. It should be noted that the "stretched state" of the diaper 1 refers to a state where the entire diaper 1 (the entire product) is stretched without being wrinkled, specifically, a state where the diaper is stretched such that the dimensions of constituent members of the diaper 1 (e.g., an absorbent main body 10, a waist member 20, or the like to be described later) match or are close to the dimensions of the members on their own.

The diaper 1 is a disposable diaper having an underpants shape in a natural state, and in the underpants-shaped state in FIG. 1, the diaper 1 has a vertical direction, a lateral direction, and a front-back direction that intersect with each other and has a waist opening BH and a pair of leg openings LH and LH. The upper side in the vertical direction corresponds to the waist opening BH side, and the lower side corresponds to the crotch side. In addition, the front side in the front-back direction corresponds to the wearer's front side, and the back side corresponds to the wearer's back side. Further, in an unfolded state in FIG. 2, the diaper 1 has a longitudinal direction and a transverse direction that intersect with each other. The longitudinal direction is a direction extending along the vertical direction in FIG. 1 and corresponds to the lengthwise direction of the absorbent main body 10. The transverse direction is a direction extending along the lateral direction in FIG. 1. In addition, as shown in FIG. 3, a direction in which constituent members of the diaper 1 are overlaid is referred to as a thickness direction. In the thickness direction, the side that comes into contact with the wearer's skin is defined as the skin side, and the side opposite to the skin side is defined as the non-skin side.

The diaper 1 has a liquid-absorbent absorbent main body 10 that absorbs excrement, and a waist member 20 that is arranged on the non-skin side of the absorbent main body 10. The waist member 20 is an exterior member that constitutes the exterior of the diaper 1, and has a front waist portion 30 corresponding to the front panel of the diaper 1 and a back waist portion 40 corresponding to the back panel of the diaper **1.** That is, the diaper 1 of the first embodiment is a so-called three-piece type underpants-shaped diaper including: as a first component, an absorbent main body 10 that is applied to the wearer's crotch portion and absorbs excrement such as urine or the like; as a second component, a front waist portion 30 that covers the wearer's stomach side portion; and as a third component, a back waist portion 40 that covers the wearer's back side portion.

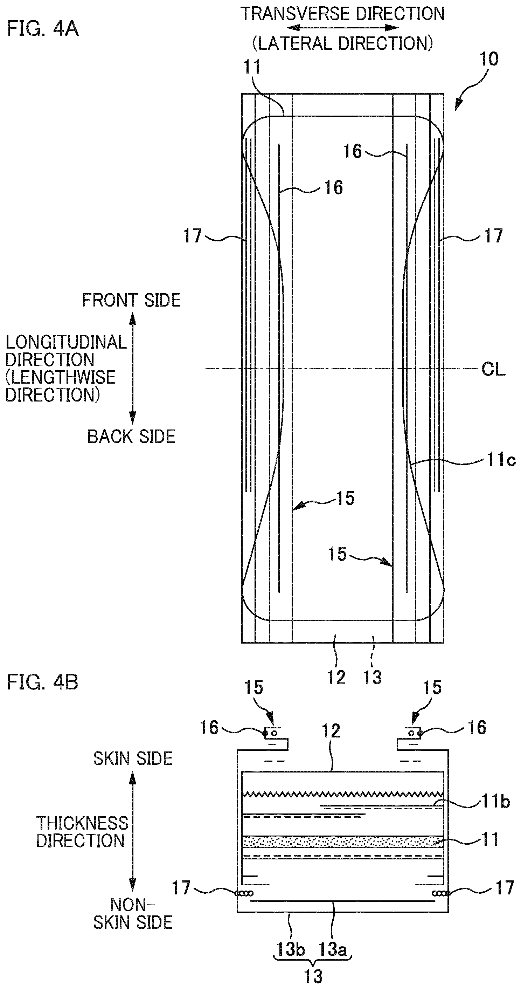

In an unfolded state in FIG. 2, in a state where the front waist portion 30 and the back waist portion 40 are arranged side by side in the longitudinal direction with a space with respect to each other, lengthwise (longitudinal) end portions 10ea and 10eb of the absorbent main body 10 are respectively joined and fixed to the skin side of the nearest waist portions 30 and 40 while the absorbent main body 10 is spanned between the front waist portion 30 and the back waist portion 40. The external shape thereof forms a substantially H shape in a plan view. Then, from this state, the absorbent main body 10 is folded one time at a fold position, which is the longitudinal central position CL. In this folded state, the front waist portion 30 and the back waist portion 40 that face each other are joined and connected to each other at two lateral side portions 30sw and 40sw to form a pair of side joining portions 50 and 50. That is, the front waist portion 30 and the back waist portion 40 are shaped into an annular shape by the pair of side joining portions 50 and 50. It should be noted that the side joining portion 50 is formed by commonly-known joining means such as welding or adhesive. Accordingly, the diaper 1 is in an underpants-shaped state in which the waist opening BH and the pair of leg openings LH and LH are formed as shown in FIG. 1. Absorbent Main body 10 FIG. 4A is a plan view of the absorbent main body 10, and FIG. 4B is a schematic cross-sectional view of the absorbent main body 10. The absorbent main body 10 has an absorbent core 11 that absorbs excreted fluids, a top sheet 12 that is arranged on the skin side in the thickness direction with respect to the absorbent core 11, and a back sheet 13 that is arranged on the non-skin side with respect to the absorbent core 11. However, the absorbent main body 10 may include other sheet members. For example, a second sheet (not shown) may be provided between the top sheet 12 and the absorbent core 11 in the thickness direction.

The absorbent core 11 is a member that absorbs and holds excreted fluid such as urine, and is formed of, for example, a liquid absorbent fiber (e.g., a pulp fiber) containing a superabsorbent polymer (SAP) . It should be noted that the outer circumferential surface of the absorbent core 11 may be covered with a liquid-permeable sheet member (core-wrapping sheet 11b) such as tissue paper or nonwoven fabric. The absorbent core 11 of the present embodiment has a narrow portion 11c, which has a narrow width in the lateral direction, between a front end and a back end in the lengthwise direction, and has a substantially hourglass shape in a plan view as shown in FIG. 4A. This narrow portion 11c is a portion sandwiched between the wearer's two legs while the diaper 1 is put on, and the lateral length thereof is small (the width is narrow), making the absorbent core 11 easier to fit to the wearer's crotch.

The top sheet 12 is a liquid-permeable sheet, and, for example, a hydrophilic air-through nonwoven fabric, spunbond nonwoven fabric, or the like may be used. In the present embodiment, as shown in FIG. 4B, two lateral side portions are folded back toward the non-skin side so as to enclose the absorbent core 11.

The back sheet 13 has a two-layer structure including a liquid-impermeable sheet 13a and an exterior sheet 13b arranged on the non-skin side of the liquid-impermeable sheet 13a. As the liquid-impermeable sheet 13a, a liquid-impermeable and moisture-permeable sheet member may be used. For example, it is possible to use a microporous breathable resin film in which a plurality of fine pores are provided in a sheet mainly made of a resin such as polyethylene or polypropylene. In the present specification, the liquid-impermeable sheet 13a will also be referred to as a "breathable film". That is, the breathable film is a sheet member having "leak-proof property" that does not allow liquids to permeate, but having "moisture permeability" or "breathability" that allows water vapor and air to permeate. On the other hand, as the exterior sheet 13b, a hydrophobic nonwoven fabric having flexibility may be used. For example, it is possible to use an air-through nonwoven fabric, a spunbond nonwoven fabric, or the like.

On two lateral side portions of the absorbent main body 10, a pair of the leak-proof wall portions 15 are respectively provided along the longitudinal direction (the lengthwise direction of the absorbent main body 10). In the present embodiment, the leak-proof wall portion 15 is formed by the above-described exterior sheet 13b. Specifically, in the lateral direction (transverse direction), a part of the exterior sheet 13b is folded toward the skin side at a plurality of locations as shown in FIG. 4B, while extending outward with respect to two end portions of the absorbent core 11, to form the pair of leak-proof wall portions 15. To the skin-side end portion (leading end portion) of each leak-proof wall portion 15, leak-proof-wall elastic members 16 such as elastic strings are attached in a state of being stretched along the longitudinal direction (the lengthwise direction of the absorbent main body 10) . While the diaper 1 is put on, the leak-proof wall portions 15 rise toward the wearer's skin side and fit to the wearer's crotch portion due to the stretchability developed by the leak-proof-wall elastic members 16.

Further, to two lateral side portions of the absorbent main body 10, leg elastic members 17 such as elastic strings are attached in a state of being stretched along the longitudinal direction (the lengthwise direction of the absorbent main body 10). While the diaper 1 is put on, the two side portions of the absorbent main body 10 contract and becomes more likely to fit around the wearer's legs due to the stretchability developed by the leg elastic members 17.

### Front Waist Portion 30

As shown in FIG. 3, the front waist portion 30 includes: a skin-side sheet 31 that is arranged farthest on the skin side in the thickness direction; a non-skin-side sheet 32 that is overlaid so as to be adjacent to the non-skin side of the skin-side sheet 31; and waist elastic members 35 that are provided between the skin-side sheet 31 and the non-skin-side sheet 32 in the thickness direction. The front waist portion 30 serving as an exterior member of the diaper 1 basically has a two-layer structure constituted by the skin-side sheet 31 and the non-skin-side sheet 32, but may partially have a configuration of three or more layers including a skin surface sheet 36 or the like described below.

The skin-side sheet 31 and the non-skin-side sheet 32 are sheet members having a rectangular shape in a plan view as shown in FIG. 2, and are formed of, for example, an SMS nonwoven fabric sheet or the like. In the diaper 1, the sheet member (nonwoven fabric sheet) that constitutes the non-skin-side sheet 32 has higher hydrophilicity than the sheet member (nonwoven fabric sheet) that constitutes the skin-side sheet 31. That is, the skin-side sheet 31 is formed of a nonwoven fabric sheet having a low hydrophilicity, and the non-skin-side sheet 32 is formed of a nonwoven fabric sheet having higher hydrophilicity than the skin-side sheet 31. Hereinafter, the nonwoven fabric that constitutes the skin-side sheet 31 will also be referred to as "hydrophobic nonwoven fabric", and the nonwoven fabric that constitutes the non-skin-side sheet 32 will also be referred to as "hydrophilic nonwoven fabric". The "hydrophilicity" of the nonwoven fabric will be described later.

Further, on the surface of the non-skin-side sheet 32, the plurality of hole portions 32h as shown in a partially enlarged view of FIG. 2 may be provided. The hole portions 32h are each a through hole that penetrates the non-skin-side sheet 32 in the thickness direction. Providing the hole portions 32h makes it possible to enhance the breathability of the front waist portion 30. Further, arranging the hole portions 32h visibly on the non-skin surface side of the front waist portion 30 makes the user more likely to invoke that the front waist portion 30 has good breathability. Each of the hole portions 32h may have, for example, a circular shape having a diameter of approximately 1 mm, but the shape and arrangement (number and pattern) of the hole portions 32h can be appropriately changed. However, in the diaper 1, the hole portions 32h does not have to be necessarily provided.

The front waist portion 30 of the present embodiment has a folded-back portion 32f in which the upper end portion (the front end portion in the longitudinal direction) of the non-skin-side sheet 32 is folded back from the non-skin side to the skin side and from the front side to the back side in the longitudinal direction. By covering a part (upper end portion) of the skin-side sheet 31 with the folded-back portion 32f, the upper end edge of the skin-side sheet 31 is prevented from biting into the wearer's skin. However, the folded-back portion 32f does not have to be necessarily provided.

Between the skin-side sheet 31 and the non-skin-side sheet 32, a plurality of waist elastic members 35 are arranged side by side in the vertical direction, and are attached in a state of being stretched in the lateral direction. The front waist portion 30 fits around the wearer's front waist due to the stretchability developed by the waist elastic member 35.

The waist elastic members 35 can be attached using an adhesive such as a hot-melt adhesive. For example, it is possible to attach the waist elastic members 35 by continuously or intermittently applying a hot-melt adhesive to each of them, stretching the waist elastic member 35 at a predetermined stretch factor, and sandwiching the waist elastic member 35 between the skin-side sheet 31 and the non-skin-side sheet 32. That is, the skin-side sheet 31 and the non-skin-side sheet 32 are joined using the adhesive with the waist elastic members 35 interposed therebetween.

Further, the front waist portion 30 may have the skin surface sheet 36. As shown in FIG. 3, the skin surface sheet 36 is a sheet member arranged so as to cover the upper end portion (the front end portion in the longitudinal direction) of the absorbent main body 10 from the skin side, and functions as a cover sheet. Accordingly, the upper end edge of the absorbent main body 10 is prevented from biting into the wearer's skin while the diaper 1 is put on. The skin surface sheet 36 is formed of, for example, a hydrophobic nonwoven fabric such as an SMS nonwoven fabric sheet or the like. It should be noted that the skin surface sheet 36 need not be necessarily provided.

In the front waist portion 30 of the present embodiment, in the case where a sheet member is provided on the skin side with respect to the skin-side sheet 31 or on the non-skin side with respect to the non-skin-side sheet 32, these sheets are arranged so as to cover only a part of the skin-side sheet 31 and the non-skin-side sheet 32. For example, the skin surface sheet 36 in FIG. 3 is provided so as to cover only a part of the skin-side sheet 31, and at least a part of the skin-side sheet 31 is in a state of being exposed to the wearer's skin side.

### Back Waist Portion 40

The back waist portion 40 has substantially the same configuration as the front waist portion 30. That is, the back waist portion 40 includes: a skin-side sheet 41 that is arranged farthest on the skin side in the thickness direction; a non-skin-side sheet 42 that is overlaid so as to be adjacent to the non-skin side of the skin-side sheet 41; and waist elastic members 45 that is provided between the skin-side sheet 41 and the non-skin-side sheet 42 in the thickness direction. Further, similar to the front waist portion 30, the back waist portion 40 may have hole portions 42h, a folded-back portion 42f, a skin surface sheet 46, and the like (see FIGS. 2 and 3). The configuration of the members is substantially the same as that of the front waist portion 30, and therefore description thereof is omitted.

On the other hand, the external shape of the back waist portion 40 is different from the external shape of the front waist portion 30. Specifically, as shown in FIG. 2, the back waist portion 40 has a buttocks cover 40b whose lower portion in the vertical direction with respect to the side joining portion 50 (side portion 40sw) has a substantially trapezoidal shape. The buttocks cover 40b is a portion whose lateral width is narrowed from the upper side toward the lower side in the vertical direction and whose outer edge is curved. By providing the buttocks cover 40b, the back waist portion 40 can widely cover the wearer's buttocks while the diaper 1 is put on.

Further, in the buttocks cover 40b, back leg elastic members 47 such as elastic strings as shown in FIG. 2 are provided. The back leg elastic members 47 are each attached between the skin-side sheet 41 and the non-skin-side sheet 42 in a state of being stretched along the outer edge portion of the buttocks cover 40b. The stretchability developed by the back leg elastic members 47 makes the buttocks cover 40b of the back waist portion 40 likely to fit to the wearer's buttocks while the diaper 1 is put on, and also makes it difficult to be turned up from the buttocks.

### Hydrophilicity of Sheet Member

Here, the hydrophilicity of the sheet member will be described. In the diaper 1, a hydrophobic nonwoven fabric is used as the skin-side sheets 31 and 41 that constitute the waist member 20 (the front waist portion 30 and the back waist portion 40) that is the exterior member, and the exterior sheet 13b of the absorbent main body 10. On the other hand, as the non-skin-side sheets 32 and 42 that constitute the waist member 20, a hydrophilic nonwoven fabric having higher hydrophilicity than a hydrophobic nonwoven fabric is used.

The hydrophilic nonwoven fabric of the present embodiment has increased hydrophilicity by undergoing a treatment for attaching a predetermined oil agent to the hydrophobic nonwoven fabric (hydrophilic treatment). As the oil agent used in the hydrophilic treatment, it is possible to use commercially available oil agents having an effect as antistatic agents for fibers, such as anionic oil agents, nonionic oil agents, and blends thereof. These oil agents are put into an oil tank and then subjected to oiling with an oiling roller or the like. As a result, the hydrophilicity of the hydrophobic nonwoven fabric can be enhanced, obtaining the hydrophilic nonwoven fabric. However, the hydrophilic nonwoven fabric may be formed by other methods. For example, a hydrophilic nonwoven fabric may be obtained by manufacturing a nonwoven fabric using highly hydrophilic fibers.

It should be noted that, in the present embodiment, it is assumed that the entire nonwoven fabric that constitutes the non-skin-side sheets 32 and 42 is subject to the hydrophilic treatment, and the hydrophilicity of the entirety of each constituent sheet member is enhanced. However, a configuration is possible in which the hydrophilicity is enhanced only in a partial region of each sheet member. For example, the sheet member may have a locally high hydrophilic portion and a locally low hydrophilic portion by undergoing a hydrophilic treatment only on the partial region of the non-skin-side sheet 32.

The hydrophilicity of a sheet member can be evaluated by measuring a contact angle when ion exchange water is brought into contact with the surface of the sheet member. Specifically, in the case where the contact angle between the hydrophilic nonwoven fabric and the ion exchange water is smaller than the contact angle between the hydrophobic nonwoven fabric and the ion exchange water, the hydrophilicity of the hydrophilic nonwoven fabric becomes higher than the hydrophilicity of the hydrophobic nonwoven fabric. For the hydrophilic nonwoven fabric (non-skin-side sheet 32 or the like) used in the present embodiment, the contact angle with the ion exchange water is preferably less than 90°, and more preferably 50° or less. On the other hand, for the hydrophobic nonwoven fabric (skin-side sheet 31), the contact angle with the ion exchange water is preferably 90° or more, and more preferably 120° or more.

The contact angle can be measured by the following method using, for example, a contact angle meter MCA-J manufactured by Kyowa Interface Science Co., Ltd. First, ion exchange water is dropped (approximately 20 picoliters) onto the surface of the fibers that constitute a sheet member (sheet to be measured), and then immediately the contact angle is measured using the contact angle meter. The measurement is performed at a plurality of places (e.g., five or more places) on the surface of the sheet to be measured, and the average value of these positions is defined as the contact angle. It should be noted that the measurement environment temperature is set to 22°C.

Alternatively, the contact angle may be measured by capturing images of the sheet to be measured onto which ion exchange water is dropped from the cross-sectional direction of the sheet to be measured, analyzing the captured image, and measuring the angle between the ion exchange water droplet and the sheet to be measured.

### Moisture Absorption and Evaporation

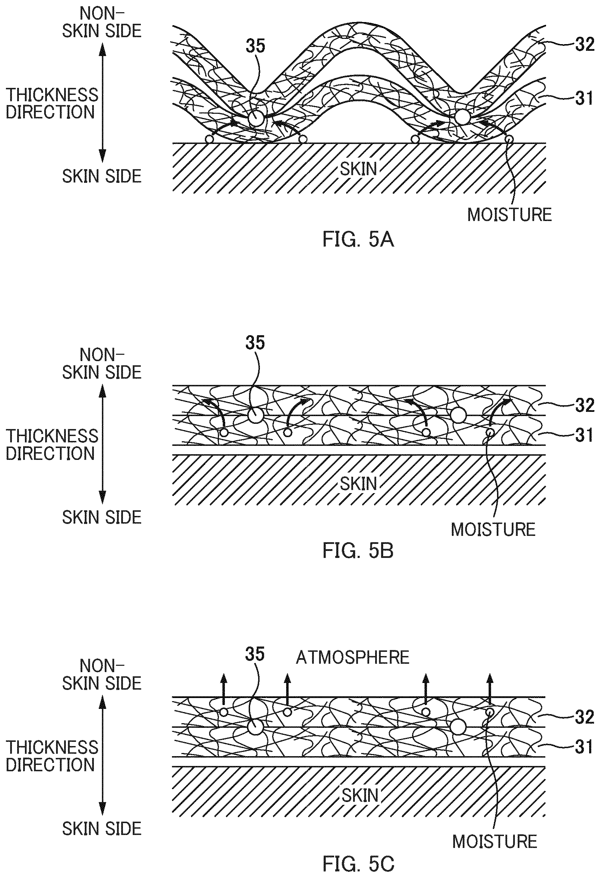

In the diaper 1, by providing the hydrophilic nonwoven fabric having a high hydrophilicity on the non-skin-side surface of the exterior member (the front waist portion 30 and the back waist portion 40), it is possible to absorb moisture such as sweat from the wearer's skin and to evaporate the absorbed moisture to the atmosphere. FIGS. 5A to 5C are diagrams illustrating the basic principle when moisture is absorbed and evaporated in the diaper 1. In FIG. 5, the cross-sections of the front waist portion 30 among the members that constitute the diaper 1 are schematically shown.

First, when the wearer puts on the diaper 1, the waist elastic members 35 provided in the front waist portion 30 contract in the lateral direction, and consequently the skin-side sheet 31 and the non-skin-side sheet 32 which are joined with the waist elastic members 35 interposed therebetween also contract in the lateral direction. Therefore, as shown in FIG. 1, a plurality of wrinkles that extend in the vertical direction are formed on the surface of the front waist portion 30. In FIG. 5A, wavy wrinkles are formed between two waist elastic members 35 and 35 which are adjacent to each other in the vertical direction by the skin-side sheet 31 and the non-skin-side sheet 32 protruding in the thickness direction. Further, in the skin-side sheet 31 (hydrophobic nonwoven fabric) that is deformed into a wavy shape as shown in FIG. 5A, a distance between fibers in the leading end portion that comes into contact with the skin is increased, and thus moisture such as sweat that is attached to the wearer's skin is likely to be absorbed in the leading end portion.

From this state, when the wearer moves the body while walking, sitting on or standing up, the contracted waist elastic members 35 are stretched, and in conjunction therewith, it generates a portion in which the skin-side sheet 31 and the non-skin-side sheet 32 are also stretched in the lateral direction. Then, in the stretched portion, the protrusions of the skin-side sheet 31 and the non-skin-side sheet 32 in the thickness direction are eliminated, and the sheets become flat along the skin as shown in FIG. 5B. As a result, the moisture absorbed by the skin-side sheet 31 is likely to be transferred to the non-skin-side sheet 32 (hydrophilic nonwoven fabric) that is overlaid adjacent to the non-skin side of the skin-side sheet 31. This is because, while the skin-side sheet 31 is formed of a hydrophobic nonwoven fabric, the non-skin-side sheet 32 is formed of a hydrophilic nonwoven fabric sheet, a difference in the magnitude of hydrophilicity (hydrophilic gradient) is thus generated between the two sheets, and this makes moisture more likely to move from the low hydrophilic skin-side sheet 31 side to the highly hydrophilic non-skin-side sheet 32 side. Therefore, moisture is less likely to be held on the skin-side sheet 31 side that is in contact with the wearer's skin, and is more likely to be held on the non-skin-side sheet 32 side that is not in contact with the wearer's skin.

The moisture held in the non-skin-side sheet 32 is evaporated into the atmosphere from the non-skin-side surface of the non-skin-side sheet 32. In the diaper 1, the non-skin-side sheet 32 is arranged on the outermost (non-skin-side) surface of the absorbent main body 10. That is, the non-skin-side surface of the non-skin-side sheet 32 serves as an interface with the atmosphere. Therefore, the moisture contained in the non-skin-side sheet 32 can be efficiently evaporated to the outside of the diaper 1 from a wide range of the non-skin-side surface. However, it is not necessary that the entire farthest-non-skin-side surface of the exterior member is formed of a hydrophilic nonwoven fabric. For example, another member different from the non-skin-side sheet 32 (e.g., a post handling tape which is used for post handling when the diaper 1 after use is disposed of) may be provided on a part of the farthest-non-skin-side surface of the exterior member.

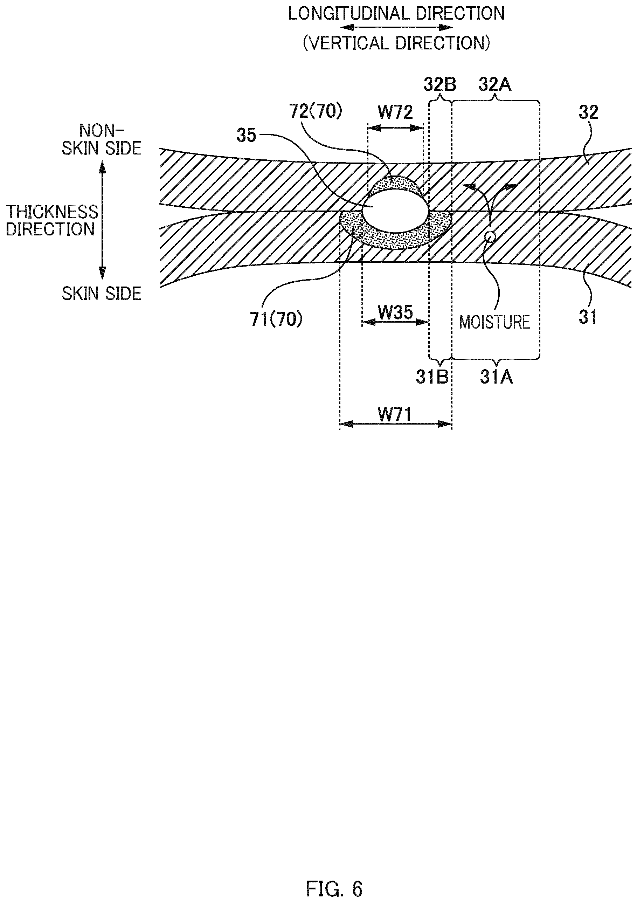

Next, the transfer of moisture from the skin-side sheet 31 (hydrophobic nonwoven fabric) to the non-skin-side sheet 32 (hydrophilic nonwoven fabric) will be described in more detail. FIG. 6 is a schematic cross-sectional view showing a vicinity of a joining portion between the skin-side sheet 31 (hydrophobic nonwoven fabric) and the non-skin-side sheet 32 (hydrophilic nonwoven fabric) in an enlarged manner.

In the diaper 1, the skin-side sheet 31 (hydrophobic nonwoven fabric) and the non-skin-side sheet 32 (hydrophilic nonwoven fabric) that constitute the front waist portion 30 (waist member 20) are joined to each other with an adhesive (e.g., a hot-melt adhesive), in a state of being overlaid in the thickness direction. Specifically, the waist elastic members 35 to which the adhesive is applied in a linear or dotted manner are sandwiched between the skin-side sheet 31 (hydrophobic nonwoven fabric) and the non-skin-side sheet 32 (hydrophilic nonwoven fabric), and thereby the skin-side sheet 31 (hydrophobic nonwoven fabric) and the non-skin-side sheet 32 (hydrophilic nonwoven fabric) are joined with the waist elastic members 35 (adhesive) interposed therebetween.

FIG. 6 shows a state where the skin-side sheet 31 (hydrophobic nonwoven fabric) and the non-skin-side sheet 32 (hydrophilic nonwoven fabric) are made adhere with an adhesive portion 70 that is formed of an adhesive. In the adhesive portion 70, a portion of the skin-side sheet 31 (hydrophobic nonwoven fabric) is referred to as a first adhesive portion 71, and a portion of the non-skin-side sheet 32 (hydrophilic nonwoven fabric) is referred to as a second adhesive portion 72. In the present embodiment, in a direction orthogonal to the direction in which the waist elastic member 35 stretches and contracts (lateral direction) (vertical direction), the width W71 of the first adhesive portion 71 is larger than the width W72 of the second adhesive portion 72 (W71 > W72). In other words, the maximum value (W72) of the adhesive width by the adhesive on the hydrophilic nonwoven fabric is narrower than the maximum value (W71) of the adhesive width by the adhesive on the hydrophobic nonwoven fabric. This is because, since the adhesive that forms the adhesive portion 70 is hydrophobic, the adhesive is more likely to conform to and to spread on the hydrophobic nonwoven fabric than on the hydrophilic nonwoven fabric. However, in the present embodiment, the adhesive does not necessarily have to be hydrophobic. For example, a configuration may be acceptable in which the width W71 of the first adhesive portion 71 on the skin side of the waist elastic members 35 is larger than the width W72 of the second adhesive portion 72 on the non-skin side thereof based on a reason such as that, in a manufacturing process of the diaper 1, the amount of the adhesive applied to the skin side of the waist elastic members 35 is larger than the amount of the adhesive applied to the non-skin side thereof.

The maximum values (W71 and W72) of the adhesive widths by the adhesive can be measured as follows. First, bamboo charcoal is applied to the non-skin-side surface of the front waist portion 30 that is in a state where the waist elastic members 35 are stretched. Then, after a predetermined time has elapsed, compressed air is blown by air blowing or the like to the bamboo charcoal attached to the non-skin-side surface of the front waist portion 30. Then, the bamboo charcoal attached to the portion of the first adhesive portion 71 remains and other bamboo charcoal is removed, and therefore the front waist portion 30 in this state is photographed from the non-skin side. Thus, the width of the portion in which the bamboo charcoal remains is measured by performing image analysis or measurement using a microscope. For example, measurement and analysis are performed at 20 times using a digital microscope VHX-7000 manufactured by Keyence Corporation. Consequently, the adhesive width W71 of the first adhesive portion 71 can be measured. Further, in the same manner for the skin side, the adhesive width W72 of the second adhesive portion 72 can be measured.

The skin-side sheet 31 (hydrophobic nonwoven fabric) and the non-skin-side sheet 32 (hydrophilic nonwoven fabric) are in close contact in the thickness direction in a region where the adhesive portion 70 is formed and in the vicinity of the region. For example, in FIG. 6, in the region of the adhesive width W71 where the first adhesive portion 71 is formed, the skin-side sheet 31 and the non-skin-side sheet 32 are made adhere to each other. A first skin-side region 31A and a first non-skin-side region 32A are in close contact with each other in the thickness direction, the first skin-side region 31A being a region that is adjacent to the outside of W71 in the skin-side sheet 31 and that has a predetermined width, the first non-skin-side region 32A being a region that faces the first skin-side region 31A in the non-skin-side sheet 32, . That is, in the first skin-side region 31A and the first non-skin-side region 32A, a space is less likely to produce between the skin-side sheet 31 (hydrophobic nonwoven fabric) and the non-skin-side sheet 32 (hydrophilic nonwoven fabric) in the thickness direction. Therefore, while the diaper 1 is put on, moisture such as sweat absorbed from the wearer's skin by the skin-side sheet 31 (hydrophobic nonwoven fabric) is likely to be transferred from the first skin-side region 31A of the skin-side sheet 31 to the first non-skin-side region 32A of the non-skin-side sheet 32 (hydrophilic nonwoven fabric) .

It should be noted that, in FIG. 6, in the skin-side sheet 31 (hydrophobic nonwoven fabric), the transfer of moisture from the skin-side sheet 31 (hydrophobic nonwoven fabric) to the non-skin-side sheet 32 (hydrophilic nonwoven fabric) is less likely to occur in the second skin-side region 31B, which is a region between the outer end of the waist elastic member 35 and the outer end of the first adhesive portion 71. This is because, in the second skin-side region 31B, the adhesive (first adhesive portion 71) is interposed between the skin-side sheet 31 and the non-skin-side sheet 32, and therefore, the movement of moisture is hindered by the adhesive.

The moisture transferred to the first non-skin-side region 32A of the non-skin-side sheet 32 (hydrophilic nonwoven fabric) is diffused in the non-skin-side sheet 32 in the planar direction (vertical direction and lateral direction). Then, the moisture is also diffused in the second non-skin-side region 32B that faces the second skin-side region 31B in the non-skin-side sheet 32. In the case where the adhesive width W72 of the second adhesive portion 72 has a width equal to or larger than the adhesive width W71 of the first adhesive portion 71, the movement of moisture is inhibited by the adhesive that forms the second adhesive portion 72, and therefore, there is a risk that the diffusible range of moisture becomes narrow in the non-skin-side sheet 32. In contrast, in the non-skin-side sheet 32 (hydrophilic nonwoven fabric) of the diaper 1, since the adhesive width W72 of the second adhesive portion 72 is narrow and the adhesive is not provided in the second non-skin-side region 32B that is adjacent to the inside of the first non-skin-side region 32A (see FIG. 6), it is less likely to prevent moisture transferred to the first non-skin-side region 32A from diffusing into the second non-skin-side region 32B. Therefore, compared with the case where the adhesive width W72 of the second adhesive portion 72 is equal to or larger than the adhesive width W71 of the first adhesive portion 71, moisture is likely to be widely diffused in the non-skin-side sheet 32. Accordingly, the evaporation of moisture from the non-skin-side surface of the non-skin-side sheet 32 to the atmosphere is promoted, making it possible to efficiently evaporate moisture in the waist member 20 (the front waist portion 30 and the back waist portion 40) of the diaper 1.

It should be noted that, even in the case where the skin-side sheet 31 (hydrophobic nonwoven fabric) and the non-skin-side sheet 32 (hydrophilic nonwoven fabric) are made adhere to each other without the waist elastic members 35, the above-described effect can be obtained. That is, even in the case where the waist elastic member 35 is not provided in FIG. 6, as long as the adhesive portions 71 and 72 are formed, the evaporation efficiency of moisture can be enhanced.

However, in the diaper 1, providing the waist elastic members 35 having stretchability in the lateral direction makes it possible to further enhance the efficiency of moisture absorption and evaporation in the front waist portion 30. For example, when the waist elastic members 35 contract in the lateral direction, the skin-side sheet 31 (hydrophobic nonwoven fabric) contracts in the lateral direction. Therefore, the skin-side sheet 31 (hydrophobic nonwoven fabric) is deformed in a wavy shape, the distance between fibers at the leading end portion that comes into contact with the skin is increased, and moisture is likely to be taken out. Then, when the waist elastic members 35 stretches in the lateral direction, the skin-side sheet 31 (hydrophobic nonwoven fabric) and the non-skin-side sheet 32 (hydrophilic nonwoven fabric) stretch in the lateral direction, and come into close contact with each other, making moisture more likely to be transferred from the skin-side sheet 31 (hydrophobic nonwoven fabric) to the non-skin-side sheet 32 (hydrophilic nonwoven fabric). Further, moisture contained in the non-skin-side sheet 32 is likely to be diffused in the lateral direction. This makes it likely to evaporate moisture from a wide range of the hydrophilic nonwoven fabric. In this way, providing the elastic members (35, 45) in the waist member 20 (30, 40) of the diaper 1 makes it possible to further enhance the evaporation efficiency of moisture.

Further, it is desirable that in the waist member 20 (30, 40) of the diaper 1, the skin-side sheets 31 and 41 (hydrophobic nonwoven fabric) have higher smoothness than the non-skin-side sheets 32 and 42 (hydrophilic nonwoven fabric) . In the case where the smoothness of the skin-side sheets 31 and 41 is low, a space between the skin-side sheets 31 and 41 and the wearer's skin is likely to be formed while the diaper 1 is put on, and there is a risk that moisture is less likely to be absorbed due to the increase of the inter-fiber distance of the skin-side sheet 31 (hydrophobic nonwoven fabric) as described in FIG. 5A. In contrast, the higher the smoothness of the skin-side sheets 31 and 41, the more likely the skin-side sheets 31 and 41 are to be in close contact with the wearer's skin while the diaper 1 is put on, and this can make it more likely to absorb moisture such as sweat from the wearer's skin.

The smoothness of the skin-side sheet 31 and the non-skin-side sheet 32 can be measured as follows. First, test pieces are taken from a region of the front waist portion 30 which stretchability by the waist elastic members 35 is exerted on, and the test pieces are attached and fixed to hook tapes such as hook-and-loop fasteners with being in a state where the waist elastic members 35 are stretched. Next, the three-dimensional shape of a region of each test piece where the adhesive portion (a portion corresponding to the adhesive portion 70 in FIG. 6) is included is photographed with a microscope or the like. For example, using a Digital Microscope VHX-7000 manufactured by Keyence Corporation, a three-dimensional shape of the object is obtained in the following way: images are taken at various heights by moving a lens up and down with respect to an object, and the images are connected by obtaining their heights at which focus is aligned at the coordinates of the screen. Then, by analyzing the cross-section of the obtained three-dimensional shape, cross-sectional waveform data in the CD direction (corresponding to the transverse direction) are acquired for a plurality of locations, and the standard deviation, maximum value, and minimum value are obtained. Among these pieces of data, the standard deviation indicates the variation of the heights of sheet members in the cross section in the CD direction. Therefore, the smaller the standard deviation, the higher the smoothness of the sheet member.

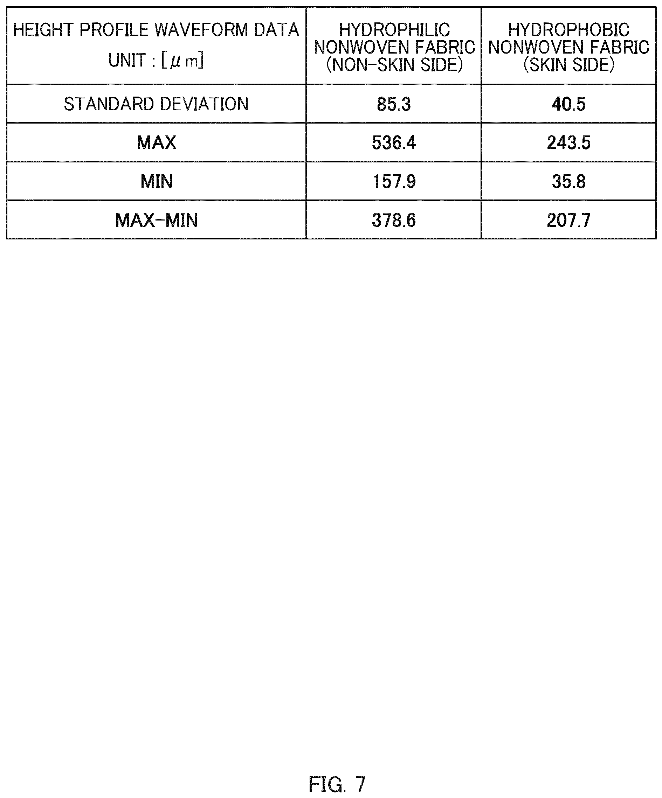

FIG. 7 is data of measurement results on a transverse cross section (CD cross section) of the waist member 20, and shows the results of photographing the CD cross section of the front waist portion 30 and measuring cross-sectional waveform data at 2000 locations. According to FIG. 7, the maximum width of the protrusions and recesses in the thickness direction of the hydrophilic nonwoven fabric (non-skin-side sheet 32) is 378.6 µm, whereas the maximum value of the protrusions and recesses in the thickness direction of the hydrophobic nonwoven fabric (skin-side sheet 31) is 207.7 µm. That is, the width of the protrusions and recesses in the thickness direction is small in the hydrophobic nonwoven fabric (skin-side sheet 31). Further, the standard deviation of the hydrophilic nonwoven fabric (non-skin-side sheet 32) is 85.3, whereas the standard deviation of the hydrophobic nonwoven fabric (skin-side sheet 31) is 40.5. That is, the variation of protrusions and recesses is small in the hydrophobic nonwoven fabric (skin-side sheet 31).

As described above, in the waist member 20 (30) of the diaper 1, the variation of the protrusions and recesses of the hydrophobic nonwoven fabric (skin-side sheet 31) is smaller than half of the variation of the hydrophilic nonwoven fabric (non-skin-side sheet 32). That is, the smoothness of the hydrophobic nonwoven fabric is two times or more greater than the smoothness of the hydrophilic nonwoven fabric. Therefore, the hydrophobic nonwoven fabric (skin-side sheet 31) is more likely to fit to the wearer's skin in a surface-to-surface manner while the diaper 1 is put on, enabling to make moisture such as sweat more likely to be absorbed.

Further, as shown in FIG. 6, the longitudinal (vertical) width W35 of the waist elastic member 35 that is in the stretched state is narrower than the adhesive width W71 of the first adhesive portion 71 (W35 < W71). In such a configuration, compared with the case where the adhesive width W71 of the first adhesive portion 71 is equal to or smaller than the width W35 of the waist elastic member 35, the region where the skin-side sheet 31 (hydrophobic nonwoven fabric) contracts accompanying with the contraction of the elastic member 35 becomes wider. Therefore, by increasing the area of the hydrophobic nonwoven fabric having high smoothness, the contact area with the skin is increased, making moisture such as sweat more likely to be absorbed. Accordingly, the skin-side sheet 31 (hydrophobic nonwoven fabric) is likely to absorb moisture such as sweat.

On the other hand, it is desirable that the longitudinal (vertical) width W35 of the waist elastic member 35 that is in the stretched state is larger than the adhesive width W72 of the second adhesive portion 72 (W35 > W72). It has been described that when diffusing moisture in the hydrophilic nonwoven fabric, the presence of the adhesive portion 70 (adhesive) makes the adhesive portion 70 likely to hinder the diffusion of moisture. In the case where the adhesive width W72 of the second adhesive portion 72 is equal to or larger than the width of the waist elastic member 35, there is a high possibility that the movement of moisture is hindered. In contrast, in the case where the adhesive width W72 of the second adhesive portion 72 is smaller than the width of the waist elastic member 35, the movement of moisture is less likely to be hindered compared with the above-described case. Then, when moisture reaches the waist elastic member 35 and is in direct contact with the surface of the waist elastic member 35, moisture is more likely to move in the lateral direction (the lengthwise direction of the waist elastic member 35) through the surface of the waist elastic member 35. That is, moisture is more likely to be further widely diffused in the non-skin-side sheet 32 (hydrophilic nonwoven fabric). Accordingly, the evaporation efficiency of moisture in the non-skin-side sheet 32 (hydrophilic nonwoven fabric) can be further enhanced.

Further, in FIG. 6A, the average density of the non-skin-side sheet 32 (hydrophilic nonwoven fabric) in the second non-skin-side region 32B that faces the second skin-side region 31B in the thickness direction is higher than the average density of the non-skin-side sheet 32 (hydrophilic nonwoven fabric) in the first non-skin-side region 32A that is adjacent to the outside of the second non-skin-side region 32B. In the non-skin-side sheet 32 (hydrophilic nonwoven fabric), in the case where the average density of the second non-skin-side region 32B is greater than the average density of the first non-skin-side region 32A, the moisture contained in the non-skin-side sheet 32 (hydrophilic nonwoven fabric) is more likely to move from the first non-skin-side region 32A to the second non-skin-side region 32B which has a high density. That is, the moisture is more likely to be diffused in the vertical direction (longitudinal direction). Further, since the second non-skin-side region 32B is provided so as to extend in the lateral direction along the waist elastic member 35, moisture is more likely to be diffused into the second non-skin-side region 32B having a high density, in the lateral direction (transverse direction). Therefore, the moisture contained in the non-skin-side sheet 32 (hydrophilic nonwoven fabric) is more likely to be widely diffused in the non-skin-side sheet 32 (hydrophilic nonwoven fabric), and the evaporation efficiency can be enhanced.

Further, in the diaper 1, the non-skin-side sheets 32 and 42 formed of a hydrophilic nonwoven fabric are arranged farthest on the non-skin side in the thickness direction of the waist member 20 (30 and 40) (see FIG. 3). That is, the hydrophilic nonwoven fabric is arranged in at least a part of the outermost layer of the waist member 20. Accordingly, at least a part of the hydrophilic nonwoven fabric serves as an interface with the atmosphere, and the moisture retained in the hydrophilic nonwoven fabric is likely to be evaporated into the atmosphere. In the case where the hydrophobic sheet member is provided overlaid on the non-skin side with respect to the hydrophilic nonwoven fabric, in the overlaid portion, there is a risk that the evaporation of moisture from the hydrophilic nonwoven fabric to the atmosphere is inhibited, making it more likely to stay in the hydrophilic nonwoven fabric. In contrast, in the diaper 1, since there is a portion in which no other member is interposed between the hydrophilic nonwoven fabric and the atmosphere, moisture can be efficiently evaporated. It should be noted that, even when another member **(e.g.,** a post handling tape for performing a post handling operation on the diaper 1) is provided on a part of the non-skin-side surface of the non-skin-side sheets 32 and 42 (hydrophilic nonwoven fabric), the above-described effect can be obtained in a region in which the other member is not provided.

At this time, it is desirable that the layer of the waist member 20 (30 and 40) which is located farthest on the non-skin side in the thickness direction is formed of one sheet of hydrophilic nonwoven fabric. Not providing extra sheet member in the waist member 20 makes it less likely to inhibit the absorption and evaporation of moisture mentioned above, and this makes it possible to more efficiently evaporate the moisture. The waist member 20 of the present embodiment partially has a three-layer structure including folded-back portions 32f and 42f, the skin surface sheets 36 and 46, and the like shown in FIG. **3****.** However, the two-layer structure in which two sheet members, that is, the skin-side sheets 31 and 41 and the non-skin-side sheets 32 and 42, are overlaid on each other in the most region of the waist member 20, and the moisture can be efficiently evaporated. Further, the hydrophilic region provided in the non-skin-side surface of the waist member 20 is formed of one sheet of hydrophilic nonwoven fabric, and therefore the hydrophilic region is formed in a wide range of the waist member 20, which makes it possible to further enhance evaporation property.

Further, as shown in FIG. 2, in the waist member 20 (30 and 40) of the diaper 1, in the region that overlap the side joining portions 50 with respect to the vertical direction, regions that are located above an upper end 11eu of the absorbent core 11 in the vertical direction are defined as upper waist regions 30U and 40U, and regions that are located below the upper end 11eu of the absorbent core 11 are defined as lower waist regions 30D and 40D. At this time, it is desirable that the ratio of the total area of the adhesive arranged in a linear or dotted manner along the waist elastic members 35 (45) in the upper waist region 30U (40U) to the area of the upper waist region 30U (40U) is larger than the ratio of the area of the adhesive arranged in a linear or dotted manner along the waist elastic members 35 (45) in the lower waist region 30D (40D) to the area of the lower waist region 30D (40D).

In the diaper 1, the vertical pitch of the waist elastic members 35 (45) arranged in the upper waist region 30U (40U) is narrower than the vertical pitch of the waist elastic members 35 (45) arranged in the lower waist region 30D (40D). That is, the ratio of the waist elastic members 35 (45) provided per unit area of the upper waist region 30U (40U) is larger than the ratio of the waist elastic members 35 (45) provided per unit area of the lower waist region 30D (40D) . Therefore, the ratio of the area of the adhesive applied continuously or intermittently along the elastic members 35 (45) is larger in the upper waist region 30U (40U) than in the lower waist region 30D (40D).

According to the above-described configuration, in the upper waist region 30U (40U), the skin-side sheet 31 (41) formed of a hydrophobic nonwoven fabric and the non-skin-side sheet 32 (42) formed of a hydrophilic nonwoven fabric are more likely to be in close contact in the thickness direction compared with the lower waist region 30D (40D). Therefore, as described in FIG. 6, the absorption and evaporation efficiency of moisture from the wearer's skin during usage of the diaper 1 is enhanced in the upper waist region 30U (40U) than in the lower waist region 30D (40D). Accordingly, in the wearer's body, in the vicinity of the waist where a large amount of sweat is produced (upper waist regions 30U and 40U), moisture can be more likely to be efficiently absorbed and evaporated.

Further, in the case where the vertical pitch of the waist elastic members 35 (45) arranged in the upper waist region 30U (40U) is narrower than the vertical pitch of the waist elastic members 35 (45) arranged in the lower waist region 30D (40D) as described above, the contractive force developed by the waist elastic members 35 (45) acting on the upper waist region 30U (40U) is likely to be larger than the contractive force developed by the waist elastic members 35 (45) acting on the lower waist region 30D (40D). In this case, the amount of lateral contraction of the hydrophobic nonwoven fabric (31 and 41) in the upper waist region 30U (40U) becomes larger than the amount of lateral contraction of the hydrophobic nonwoven fabric (31 and 41) in the lower waist region 30D (40D) . Therefore, in the upper waist region 30U (40U), the absorption of moisture (sweat) due to the capillary phenomenon described in FIG. 5A is promoted, and the moisture is more likely to be absorbed in the vicinity of the waist where a large amount of sweat is produced, enabling to make stuffiness or rashes less likely to occur while the diaper 1 is put on.

Further, on the skin side of the waist member 20 (30 and 40) of the diaper 1, the skin surface sheets 36 and 46 formed of a hydrophobic nonwoven fabric are provided so as to straddle upper ends 10ea and 10eb of the absorbent main body 10 in the vertical direction (see FIGS. 2 and 3). While the diaper 1 is put on, the top sheet 12 is more likely to be pressed against the wearer's skin at edge portions of the upper ends 10ea and 10eb of the absorbent main body 10. Since the top sheet 12 is a hydrophilic nonwoven fabric, moisture such as sweat is likely to be absorbed. Accordingly, when the top sheet 12 containing moisture comes into contact with the wearer's skin by the upper ends 10ea and 10eb, there is a case where the wearer misinterprets that urine absorbed by the absorbent main body is attached to the skin (so-called rewet), feeling discomfort. Therefore, in the diaper 1, by arranging the skin surface sheets 36 and 46 (hydrophobic nonwoven fabric) so as to straddle the upper ends 10ea and 10eb of the absorbent main body 10, it suppresses the contact of the top sheet 12 containing moisture (sweat) with the wearer's skin at the upper ends 10ea and 10eb. This can make the wearer less likely to feel discomfort.

Further, the skin surface sheets 36 and 46 are each provided so as to have a portion that overlaps the adhesive portion 70 (adhesive) of the waist member 20 (30 and 40) when viewed in the thickness direction (see FIG. 3 and the like). In other words, there is a portion in which at least some of the waist elastic members 35 (45) to which the adhesive is applied and the skin surface sheets 36 and 46 overlap when viewed in the thickness direction. As described above, there is a case where moisture such as sweat absorbed by the non-skin-side sheets 32 and 42 of the waist member 20 be diffused through the waist elastic members 35 and **45.** In such a case, in a portion where the skin surface sheet 36 (46) is provided on the non-skin side of the waist elastic members 35 (45) (adhesive portion 70), it prevents the moisture diffused through the waist elastic members 35 (45) from being transferred to the skin side of the wearer (rewetting), and this can make the wearer less likely to feel discomfort.

Further, the skin surface sheets 36 and 46 are provided spaced apart from the upper end of the waist member 20 by a predetermined distance in the vertical direction of the diaper 1 (see FIG. 3). That is, a predetermined distance is provided between the upper end of the waist member 20 and each of the upper ends of the skin surface sheets 36 and **46.** In the region where the skin surface sheet 36 (46) is provided on the skin-side surface of the waist member 20, compared with the region where the skin surface sheet 36 (46) is not provided, the transfer of moisture from the skin side to the non-skin side is less likely to occur. This is because, by overlaying two layers of hydrophobic nonwoven fabric, namely the skin-side sheet 31 (41) and the skin surface sheet 36 (46), on the skin side of the non-skin-side sheet 32 (42) (hydrophilic nonwoven fabric), moisture is less likely to be transferred between the two layers of the hydrophobic nonwoven fabric. Therefore, in the case where the skin surface sheets 36 and 46 are provided in the vicinity of the upper end portion of the waist member 20, there is a risk that evaporation of moisture (sweat) is less likely to occur in the vicinity of the waist where a large amount of sweat is produced, making the vicinity of the waist moist to cause an discomfort feeling to the wearer. In contrast, in the present embodiment, since the skin surface sheets 36 and 46 are provided spaced apart from the upper end of the waist member 20, moisture (sweat) can be efficiently absorbed in the upper end region (in the vicinity of the waist) of the waist member 20 and evaporated toward the non-skin side. Accordingly, the wearer can be less likely to feel discomfort while the diaper is put on.

Further, in the back waist portion 40 of the diaper 1, the back leg elastic members 47 are provided along the outer edge of the buttocks cover 40b, and the back leg elastic members 47 have portions that intersect the waist elastic members 45, in two lateral side portions 40sw and 40sw of the back waist portion 40 (that is, the side joining portions 50 and 50). In the waist member 20 of the diaper 1, in the side joining portions 50, the side portions 30sw of the front waist portion 30 and the side portions 40sw of the back waist portion 40 are joined in a state where they are overlaid in the thickness direction. This makes the side joining portions 50 be regions where a large number of materials (sheet members) are overlaid on each other and where stuffiness is more likely to occur. Therefore, in the present embodiment, the waist elastic member 45 and the back leg elastic member 47 intersect at least in a part of the side joining portions 50, and the contractive force generated by the two types of elastic member 45 and 47 acts on the regions. Accordingly, the contractive force in the side joining portions 50 is increased, the hydrophobic nonwoven fabric is deformed in a wavy shape, increasing the distance between the fibers at the leading end portion that comes into contact with the skin. This makes it more likely to take moisture and to transfer liquid to the hydrophilic nonwoven fabric which is in close contact. Therefore, stuffiness is suppressed in the side joining portions 50, and the wearer can be less likely to feel discomfort.

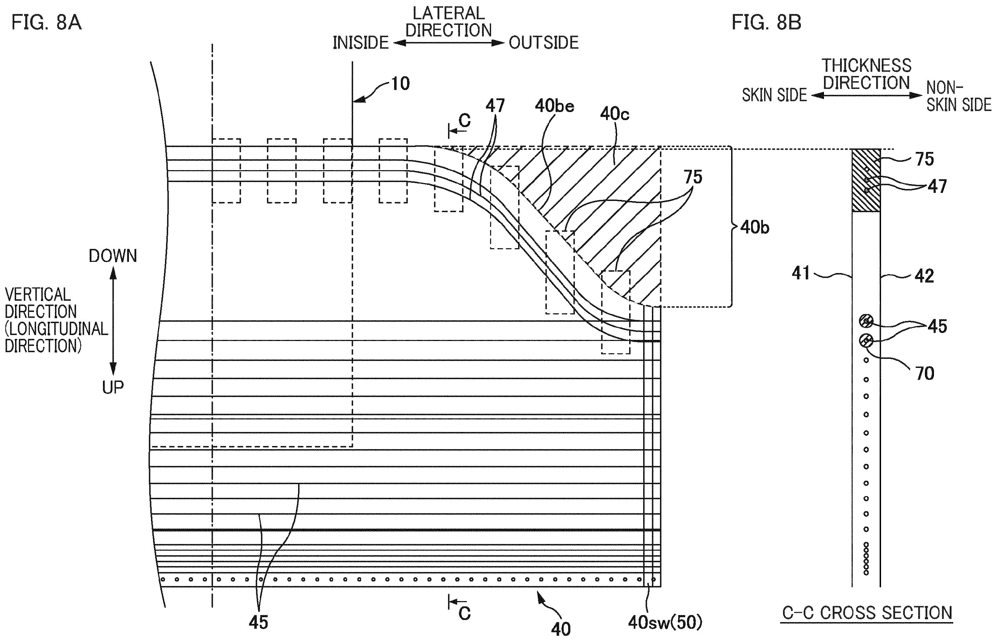

Further, in the buttocks cover 40b of the back waist portion 40, the skin-side sheet 41 (hydrophobic nonwoven fabric) and the non-skin-side sheet 42 (hydrophilic nonwoven fabric) are compressed in the thickness direction at an outer edge 40be thereof. FIGS. 8A and 8B are diagrams illustrating the configuration of the buttocks cover 40b of the back waist portion 40. FIG. 8A is a plan view showing a part (a region on the one side in the lateral direction) of the back waist portion 40 which is in a stretched and unfolded state, and FIG. 8B is a schematic cross-sectional view taken along a line C-C in FIG. 8A.

When the back waist portion 40 is manufactured in the manufacturing process of the diaper 1, first, sheet members (materials) that constitute the skin-side sheet 41 and the non-skin-side sheet 42 are prepared. Although detailed description is omitted, the sheet members are fed as a continuous body having a band-like shape, and while being transported in the direction along the lateral direction of FIG. 8A, the sheet members are joined to each other in a state where the waist elastic members 45 and the back leg elastic members 47 are sandwiched between the sheet members in the thickness direction. At this time, the adhesive portions 70 are formed in the waist elastic members 45 by applying an adhesive (e.g., a hot-melt adhesive) thereto, and the adhesive portions 70 make the skin-side sheet 41 and the non-skin-side sheet 42 join, consequently attaching the waist elastic members 45 to the sheet members.

On the other hand, the back leg elastic members 47 are attached by the buttocks-side adhesive portions 75, which are formed on at least one of the skin-side sheet 41 and the non-skin-side sheet 42. The buttocks-side adhesive portions 75 are a plurality of rectangular portions each of which is surrounded by a dotted line in FIG. 8A, and are portions to which an adhesive such as a hot-melt adhesive is applied along a region where the back leg elastic members 47 are arranged. After the buttocks-side adhesive portions 75 are formed, by sandwiching the leg elastic members 47 between the skin-side sheet 41 and the non-skin-side sheet 42 in a state where the leg elastic members 47 are stretched, the skin-side sheet 41 and the non-skin-side sheet 42 are joined to each other and the back leg elastic members 47 are attached to the sheet members.

Thereafter, the band-like continuous bodies of the skin-side sheet 41 and the non-skin-side sheet 42 are cut at a predetermined length in the direction in which the continuous bodies are transported (corresponding to the lateral direction), thereby forming individual pieces of the back waist portion 40. Then, by cutting off a region 40c (two end portions in the lateral direction and a lower end region in the vertical direction) indicated by hatched lines in FIG. 8A, the buttocks cover 40b in which the outer edge 40be is curved is formed. The cutting of the region 40c is performed, for example, by sandwiching the skin-side sheet 41 and the non-skin-side sheet 42 which are overlaid in the thickness direction with a cutter in the thickness direction and cutting them along the outer edge 40be.

Since the buttocks cover 40b of the back waist portion 40, which has been formed through such a step, is sandwiched by a cutter at the time of cutting off the region 40c, the buttocks cover 40b is in a state where the outer edge 40be is compressed in the thickness direction. That is, in the outer edge 40be of the buttocks cover 40b, the skin-side sheet 41 (hydrophobic nonwoven fabric) and the non-skin-side sheet 42 (hydrophilic nonwoven fabric) are compressed in the thickness direction, and are in a state of being in close contact with each other. Accordingly, the transfer of moisture from the hydrophobic nonwoven fabric to the hydrophilic nonwoven fabric is likely to occur, and in the buttocks cover 40b of the back waist portion 40, the function of absorbing and evaporating moisture such as sweat can be enhanced.

Further, the region along the outer edge 40be of the buttocks cover 40b has a portion where the skin-side sheet 41 (hydrophobic nonwoven fabric) and the non-skin-side sheet 42 (hydrophilic nonwoven fabric) are joined by the buttocks-side adhesive portion 75 and a portion where the skin-side sheet 41 and the non-skin-side sheet 42 are not joined. That is, in a region where the outer edge 40be and the buttocks-side adhesive portion 75 overlap, the skin-side sheet 41 and the non-skin-side sheet 42 are joined to each other in a region on and inside the outer edge 40be (see FIGS. 8A and 8B). On the other hand, in a region where the outer edge 40be and the buttocks-side adhesive portion 75 do not overlap, the skin-side sheet 41 and the non-skin-side sheet 42 are not joined in the region on and inside the outer edge 40be (as described above, the outer edge 40be itself is compressed). In the region where the skin-side sheet 41 and the non-skin-side sheet 42 are joined, the buttocks cover 40b has a sufficient strength, and the skin-side sheet 41 and the non-skin-side sheet 42 are less likely to be peeled off even when the wearer moves the body. On the other hand, in a region where the skin-side sheet 41 and the non-skin-side sheet 42 are not joined, the buttocks cover 40b has flexibility. Therefore, the buttocks cover is likely to fit to the wearer's body, and the wearer can feel a soft texture. As described above, in the buttocks cover 40b of the diaper 1, moisture absorption and evaporation property in the buttocks can be enhanced while securing strength and flexibility.

### Second Embodiment

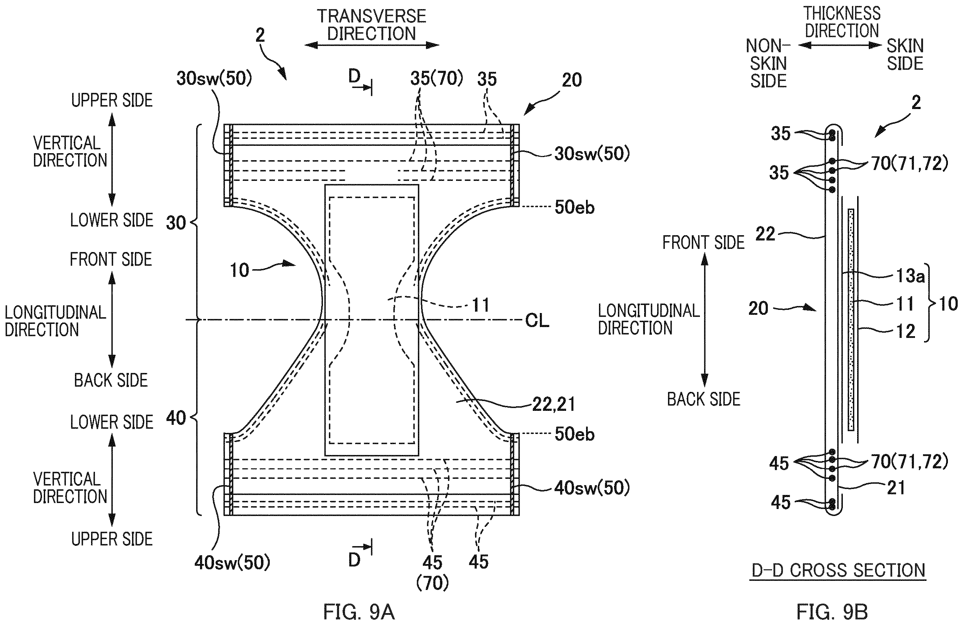

In a second embodiment, an underpants-shaped diaper 2 (hereinafter, also referred to as a "diaper 2") having a configuration partially different from that of the first embodiment will be described. FIG. 9A is a plan view of the diaper 2 in the unfolded and stretched state. FIG. 9B is a schematic cross-sectional view taken along a line D-D in FIG. 9A. It should be noted that the directions (e.g., the longitudinal direction, the transverse direction, or the like) in FIGS. 9A and 9B is the same as the directions defined in the first embodiment.

The diaper 2 of the second embodiment has the liquid-absorbent absorbent main body 10 and the waist member 20 that is joined to the non-skin side of the absorbent main body 10 as an exterior member. Further, as shown in FIGS. 9A and 9B, the waist member 20 of the diaper 2 is integrally constituted so as to be continuous in the longitudinal direction (the lengthwise direction of the absorbent main body 10). That is, the diaper 2 is a so-called two-piece type disposable diaper formed of two parts: the absorbent main body 10 and an exterior member (waist member 20). Hereinafter, in the exterior member (waist member 20) of the diaper 2, a portion located on the front side with respect to the longitudinal central position CL is defined as the front waist portion 30, and a portion located on the back side with respect to the central position CL is defined as the back waist portion 40 (see FIG. 9A).

When shaping the diaper 2 in the unfolded state in FIG. 9A into an underpants shape, the absorbent main body 10 and the waist member 20 are folded one time at a fold position, which is the longitudinal central position CL. In this folded state, the front waist portion 30 and the back waist portion 40 that face each other are joined and connected to each other at two lateral side portions 30sw and 40sw to form the pair of side joining portions 50 and 50. Accordingly, similar to the diaper 1 in FIG. 1, the diaper 2 is in an underpants-shaped state having a waist opening BH and a pair of leg openings LH and LH.

In the diaper 2, the basic configuration and function of the absorbent main body 10 are substantially the same as those of the absorbent main body 10 of the diaper 1 of the first embodiment, and therefore description thereof is omitted. However, the back sheet 13 of the diaper 2 may include only the liquid-impermeable sheet 13a (breathable film) and need not include the exterior sheet 13b. This is because in the diaper 2, the waist member 20 is provided on the entire non-skin-side surface of the absorbent main body 10, and the waist member 20 functions as an exterior sheet of the absorbent main body 10. Therefore, as the back sheet 13 of the absorbent main body 10 of the diaper 2, a sheet member corresponding to the exterior sheet 13b of the diaper 1 need not be provided (see FIG. 9B).

The waist member 20 includes: a skin-side sheet 21; a non-skin-side sheet 22 that is overlaid to be adjacent to the non-skin side of the skin-side sheet 21; and waist elastic members 35 and 45 that are arranged between the skin-side sheet 21 and the non-skin-side sheet 22 in the thickness direction (see FIG. 9B). The skin-side sheet 21 is a hydrophobic nonwoven fabric sheet similar to the skin-side sheets 31 and 41 of the diaper **1.** The non-skin-side sheet 22 is a hydrophilic nonwoven fabric sheet similar to the non-skin-side sheets 32 and 42 of the diaper **1.** That is, in the diaper 2, the non-skin-side sheet 22 is a nonwoven fabric sheet having higher hydrophilicity than the skin-side sheet 21. Then, similar to the diaper 1, the waist elastic members 35 and 45 are formed of elastic strings or the like, and are attached between the skin-side sheet 21 and the non-skin-side sheet 22 by the adhesive portion 70 (first adhesive portion 71 and the second adhesive portion 72) formed of an adhesive, in a state of being stretched in the lateral direction.

Also in the diaper 2 of the second embodiment, the same effect as that of the diaper 1 can be obtained. That is, since the skin-side sheet 21 (hydrophobic nonwoven fabric) and the non-skin-side sheet 22 (hydrophilic nonwoven fabric) are in close contact with each other via the adhesive portion 70, the transfer of moisture from the skin side to the non-skin side is promoted. Further, in the non-skin-side sheet 22 (hydrophilic nonwoven fabric), compared with the case where the adhesive width W72 of the second adhesive portion 72 is equal to or larger than the adhesive width W71 of the first adhesive portion 71, when the adhesive width W72 of the second adhesive portion 72 is narrow, moisture is likely to be widely diffused in the non-skin-side sheet 22 (see FIG. 6). Accordingly, the moisture absorbed from the skin-side sheet 21 is more likely to be evaporated from the non-skin-side sheet 22 to the atmosphere, making it possible to efficiently evaporate the moisture in the exterior members (the front waist portion 30 and the back waist portion 40) of the diaper 2.

### Third Embodiment

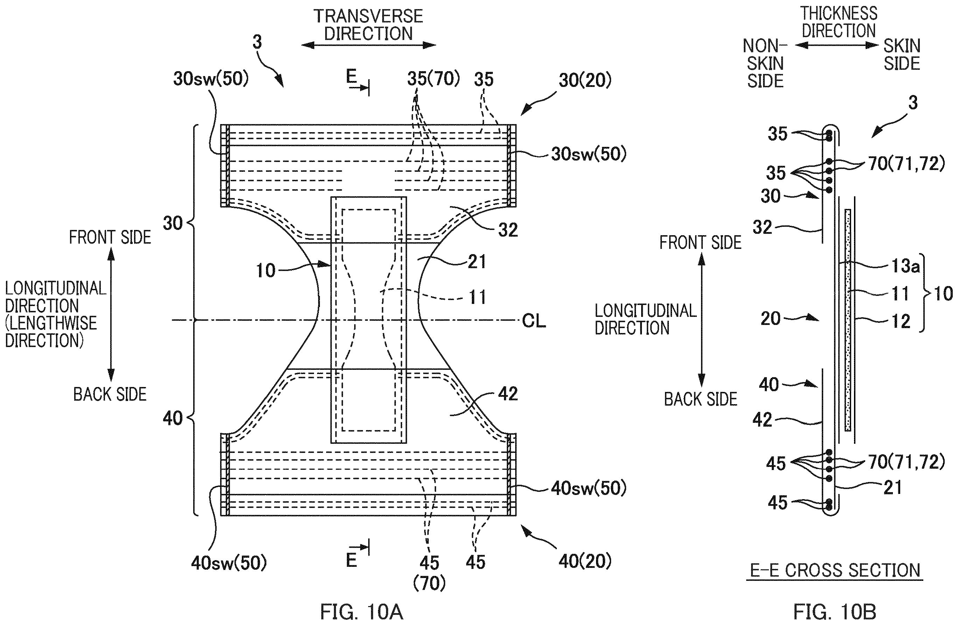

In a third embodiment, an underpants-shaped diaper 3 (hereinafter, also referred to as a "diaper 3") having a configuration partially different from that of the above embodiments will be described. FIG. 10A is a plan view of the diaper 3 in the unfolded and stretched state. FIG. 10B is a schematic cross-sectional view taken along a line E-E in FIG. 10A. It should be noted that the directions (e.g., the longitudinal direction, the transverse direction, or the like) in FIGS. 10A and 10B is the same as the directions in the first embodiment.

The diaper 3 of the third embodiment has the liquid-absorbent absorbent main body 10 and the waist member 20 that is joined to the non-skin side of the absorbent main body 10 as an exterior member. As shown in FIGS. 10A and 10B, the waist member 20 of the diaper 3 is integrally constituted so as to be continuous in the longitudinal direction (the lengthwise direction of the absorbent main body 10) . That is, the skin-side sheet 21 is configured as an integral sheet member that is continuous from an end portion located on the one side to an end portion located on the other side in the longitudinal direction. On the other hand, the non-skin-side sheet 32 arranged on the longitudinal front side (front non-skin-side sheet) and the non-skin-side sheet 42 arranged on the longitudinal back side (back non-skin-side sheet) are configured as different sheet members that are non-continuous in the longitudinal direction. A diaper having such a structure will also be referred to as a simple three-piece type disposable diaper. Further, hereinafter, in the exterior member (waist member 20) of the diaper 3, a portion located on the front side with respect to the longitudinal central position CL is defined as the front waist portion 30, and a portion located on the back side with respect to the central position CL is defined to as the back waist portion 40 (see FIG. 10A).

When shaping the diaper 3 in the unfolded state in FIG. 10A into an underpants shape, the absorbent main body 10 and the waist member 20 are folded one time at a fold position, which is the longitudinal central position CL.

In this folded state, the front waist portion 30 and the back waist portion 40 that face each other are joined and connected to each other at two lateral side portions 30sw and 40sw to form the pair of side joining portions 50 and 50. Accordingly, similar to the diaper 1 in FIG. 1, the diaper 3 is in an underpants-shaped state having a waist opening BH and a pair of leg openings LH and LH.

In the diaper 3, the basic configuration and function of the absorbent main body 10 are substantially the same as those of the absorbent main body 10 of the diaper 2 of the second embodiment, and therefore description thereof is omitted.

In the exterior member (waist member 20) of the diaper 3, the front waist portion 30 includes: the single skin-side sheet 21 that is located on the skin side in the thickness direction and extends from one end side (front side) to the other end side (back side) in the longitudinal direction; the non-skin-side sheet 32 that is located on the front side in the longitudinal direction and is overlaid to be adjacent to the non-skin side of the skin-side sheet 21; and the waist elastic members 35 that are arranged between the skin-side sheet 21 and the non-skin-side sheet 32 in the thickness direction. The back waist portion 40 includes: the skin-side sheet 21 that is common to the front waist portion 30; the non-skin-side sheet 42 that is located on the back side in the longitudinal direction and is overlaid to be adjacent to the non-skin side of the skin-side sheet 21; and the waist elastic members 45 that are arranged between the skin-side sheet 21 and the non-skin-side sheet 42 in the thickness direction.

The skin-side sheet 21 is a hydrophobic nonwoven fabric sheet similar to the skin-side sheets 31 and 41 of the diaper **1.** The non-skin-side sheets 32 and 42 are hydrophilic nonwoven fabric sheets similar to the non-skin-side sheets 32 and 42 of the diaper **1.** That is, in the diaper 3, the non-skin-side sheets 32 and 42 are nonwoven fabric sheets having higher hydrophilicity than the skin-side sheet 21. Then, similar to the diaper 1, the waist elastic members 35 and 45 are formed of elastic strings or the like, and are attached between the skin-side sheet 21 and the non-skin-side sheets 32 and 42 by the adhesive portion 70 (first adhesive portion 71 and the second adhesive portion 72) formed of an adhesive, in a state of being stretched in the lateral direction.

Also in the diaper 3 of the third embodiment, the same effect as that of the diaper 1 can be obtained. That is, since the skin-side sheet 21 (hydrophobic nonwoven fabric) and the non-skin-side sheets 32 and 42 (hydrophilic nonwoven fabrics) are in close contact with each other via the adhesive portion 70, the transfer of moisture from the skin side to the non-skin side is promoted. Further, in the non-skin-side sheets 32 and 42 (hydrophilic nonwoven fabrics), compared with the case where the adhesive width W72 of the second adhesive portion 72 is equal to or larger than the adhesive width W71 of the first adhesive portion 71, when the adhesive width W72 of the second adhesive portion 72 is narrow, moisture is likely to be widely diffused in the non-skin-side sheets 32 and 42 (see FIG. 6). Accordingly, the moisture absorbed from the skin-side sheet 21 is more likely to be evaporated from the non-skin-side sheets 32 and 42 to the atmosphere, making it possible to efficiently evaporate the moisture in the exterior members (the front waist portion 30 and the back waist portion 40) of the diaper 3.

### Other Embodiments

Although the above embodiments of the present invention have been described, but the above-described embodiments are intended to facilitate the understanding of the present invention and are not intended to limit the interpretation of the present invention. In addition, the present invention can be modified or improved within the scope of the gist of the present invention, and it is needless to say that equivalents thereof are included in the present invention defined by the claims.

### REFERENCE SIGNS LIST

1: diaper (absorbent article) (first embodiment),
2: diaper (absorbent article) (second embodiment),
3: diaper (absorbent article) (third embodiment),
10: absorbent main body,
11: absorbent core, 11b: core-wrapping sheet, 11c: narrow portion,
12: top sheet,
13: back sheet,
13a: liquid-impermeable sheet (breathable film), 13b: exterior sheet,
15: leak-proof wall portion, 16: leak-proof wall elastic member,
17: leg elastic member,
18: side sheet, 19: back elastic member,
20: waist member,
21: skin-side sheet (hydrophobic non-woven fabric),
22: non-skin-side sheet (hydrophilic non-woven fabric),
30: front waist portion,
30U: upper waist region, 30D: lower waist region, 30sw: side portion,
31: skin-side sheet (hydrophobic nonwoven fabric),
31A: first skin-side region, 31B: second skin-side region,
32: non-skin-side sheet (hydrophilic nonwoven fabric),
32A: first non-skin-side region, 32B: second non-skin-side region,
32f: folded-back portion, 32h: hole portion,
35: waist elastic member,
36: skin surface sheet,
40: back waist portion,
40U: upper waist region, 40D: lower waist region,
40b: buttocks cover, 40be: outer edge, 40sw: side portion,
41: skin-side sheet (hydrophobic nonwoven fabric),
42: non-skin-side sheet (hydrophilic nonwoven fabric), 42f: folded-back portion, 42h: hole portion,
45: waist elastic member,
46: skin surface sheet, 47: back leg elastic member,
50: side joining portion,
70: adhesive portion,
71: first adhesive portion, 72: second adhesive portion, 75: buttocks-side adhesive portion,
BH: waist opening, LH: leg opening,
CL: central position (longitudinal direction, lengthwise direction),
W71: adhesive width (first adhesive portion), W72: adhesive width (second adhesive portion)

## Claims

1. An absorbent article (1, 2, 3) comprising:
a liquid-absorbent absorbent main body (10); and
a waist member (20) that is provided on a non-skin side with respect to the absorbent main body,
the waist member (20) having a hydrophobic nonwoven fabric (21, 31, 41) and a hydrophilic nonwoven fabric (22, 32, 42) at least in a partial region,
the hydrophilic nonwoven fabric (22, 32, 42)
that is overlaid to be adjacent to a non-skin side of the hydrophobic nonwoven fabric (21, 31, 41) and
that has higher hydrophilicity than the hydrophobic nonwoven fabric (21, 31, 41),
the waist member (20) having a portion where the hydrophilic nonwoven fabric (22, 32, 42) and the hydrophobic nonwoven fabric (21, 31, 42) are made adhere to each other with an adhesive,
a maximum adhesive width (W72) by the adhesive in the hydrophilic nonwoven fabric (22, 32, 42) being narrower than a maximum adhesive width (W71) by the adhesive in the hydrophobic nonwoven fabric (21, 31, 41), wherein
the absorbent article (1, 2, 3) has a vertical direction and a lateral direction that intersect with each other,
the waist member (20) has a waist elastic member (35, 45) that stretches and contracts in the lateral direction,
the adhesive is provided along the waist elastic member (35, 45), and
a width of a cross section of the waist elastic member (35, 45) that is in a state of being stretched in the lateral direction is narrower than the maximum adhesive width (W71) by the adhesive on the hydrophobic nonwoven fabric (21, 31, 41), and wherein
the waist member (20) has
a second skin-side region (31B) that is a region between an outer end of the adhesive provided in the hydrophobic nonwoven fabric (21, 31, 41) and an outer end of the waist elastic member (35, 45),
a second non-skin-side region (32B) that is a region facing the second skin-side region (31B) in the hydrophilic nonwoven fabric (22, 32, 42), and
a first non-skin-side region (32A) that is a region having a predetermined width and adjacent to an outside of the second non-skin-side region (32B) in the hydrophilic nonwoven fabric (22, 32, 42), and
an average density of the hydrophilic nonwoven fabric (22, 32, 42) in the second non-skin-side region (32B) is greater than an average density of the hydrophilic nonwoven fabric (22, 32, 42) in the first non-skin-side region (32A).

2. The absorbent article (1, 2, 3) according to claim 1, wherein
at least a part of the hydrophilic nonwoven fabric (22, 32, 42) is arranged farthest on a non-skin side of the waist member (20).

3. The absorbent article (1, 2, 3) according to any one of claims 1 or 2, wherein
the waist member (20) has a front waist portion (30) and a back waist portion (40), and
a farthest-on-non-skin-side layer of each of the front waist portion (30) and the back waist portion (40) is formed of one sheet of hydrophilic nonwoven fabric.

4. The absorbent article (1) according to any one of claims 1 to 3, wherein
the absorbent article (1) has a vertical direction and a lateral direction that intersect with each other,
the absorbent main body (10) has an absorbent core (11),
the waist member (20) has a front waist portion (30) and a back waist portion (40),
the front waist portion (30) and the back waist portion (40) are annularly joined by a pair of side joining portions (50) that are provided in two end portions in the lateral direction, and
concerning an upper waist region (30U, 40U) that overlaps the side joining portion (50) of the waist member (20) with respect to the vertical direction and that is located above an upper end of the absorbent core (11) in the vertical direction,
concerning a lower waist region (30D, 40D) that overlaps the side joining portion (50) of the waist member (20) with respect to the vertical direction and that is located below the upper end of the absorbent core (11) in the vertical direction,
a ratio of a total area of the adhesive arranged in the upper waist region (30U, 40U) in a linear or dotted manner to an area of the upper waist region (30U, 40U) is larger than a ratio of a total area of the adhesive arranged in the lower waist region (30D, 40D) in a linear or dotted manner to an area of the lower waist region (30D, 40D).

5. The absorbent article (1) according to claim 4, wherein
the waist member (20) has a plurality of waist elastic members (35, 45) that stretch and contract in the lateral direction, and a vertical pitch between the plurality of waist elastic members (35, 45) that are provided in the upper waist region (30U, 40U) is narrower than a vertical pitch between the plurality of waist elastic members (35, 45) that are provided in the lower waist region (30D, 40D) .

6. The absorbent article (1) according to claim 4 or 5, wherein
the back waist portion (40) includes a buttocks cover (40b) below lower ends of the side joining portions (50),
the buttocks cover (40b) having a lateral width that is narrowed from an upper side to a lower side in the vertical direction,
the back waist portion (40) includes a back leg elastic member (47) and a waist elastic member (45),
the back leg elastic member (47) stretching and contracting along an end edge portion of the buttocks cover (40b),
the waist elastic member (45) stretching and contracting along the lateral direction, and
the back leg elastic member (47) has a portion that intersects the waist elastic member (45), in the side joining portion (50).

7. The absorbent article (1) according to claim 6, wherein
in an outer edge portion (40be) of the buttocks cover (40b), the absorbent article (1) has a portion in which the hydrophilic nonwoven fabric (42) and the hydrophobic nonwoven fabric (41) are compressed.

8. The absorbent article (1) according to claim 6 or 7, wherein
in an outer edge portion (40be) of the buttocks cover (40b), the absorbent article (1) has
a portion where the hydrophilic nonwoven fabric (42) and the hydrophobic nonwoven (41) fabric are joined by an adhesive and
a portion where the hydrophilic nonwoven fabric (42) and the hydrophobic nonwoven fabric (41) are not joined.

9. The absorbent article (1) according to any one of claims 1 to 8,
wherein
the absorbent article (1) has a vertical direction and a lateral direction that intersect with each other, and
a skin surface sheet (36, 46) formed of a hydrophobic nonwoven fabric is provided on a skin side of the waist member (20), so as to straddle an upper end of the absorbent main body (10) in the vertical direction.

10. The absorbent article (1) according to claim 9, wherein
the absorbent article (1) has a portion in which the skin surface sheet (36, 46) and the adhesive overlap when viewed in a thickness direction of the waist member (20).

11. The absorbent article (1, 2, 3) according to claim 9 or 10, wherein
the skin surface sheet (36, 46) is provided spaced apart from an upper end of the waist member (20) by a predetermined distance in the vertical direction.

## Patentansprüche

1. Absorbierender Artikel (1, 2, 3), der Folgendes umfasst:
einen flüssigkeitsabsorbierenden absorbierenden Hauptkörper (10); und
ein Taillenelement (20), das auf einer Nicht-Hautseite in Bezug auf den absorbierenden Hauptkörper bereitgestellt ist,
wobei das Taillenelement (20) einen hydrophoben Vliesstoff (21, 31, 41) und einen hydrophilen Vliesstoff (22, 32, 42) zumindest in einem Teilbereich aufweist,
der hydrophile Vliesstoff (22, 32, 42),
der angrenzend an eine Nicht-Hautseite des hydrophoben Vliesstoffs (21, 31, 41) überlagert und
der eine höhere Hydrophilie aufweist als der hydrophobe Vliesstoff (21, 31, 41),
wobei das Taillenelement (20) einen Teil aufweist, wo der hydrophile Vliesstoff (22, 32, 42) und der hydrophobe Vliesstoff (21, 31, 41) mit einem Haftmittel aneinander haften,
eine maximale Haftmittelbreite (W72) durch das Haftmittel in dem hydrophilen Vliesstoff (22, 32, 42) schmaler ist als eine maximale Haftmittelbreite (W71) durch das Haftmittel in dem hydrophoben Vliesstoff (21, 31, 41), wobei
der absorbierende Artikel (1, 2, 3) eine vertikale Richtung und eine laterale Richtung aufweist, die einander schneiden,
das Taillenelement (20) ein elastisches Taillenelement (35, 45) aufweist, das sich in der lateralen Richtung dehnt und zusammenzieht,
das Haftmittel entlang des elastischen Taillenelements (35, 45) bereitgestellt ist und
eine Breite eines Querschnitts des elastischen Taillenelements (35, 45), das in einem in der lateralen Richtung gedehnten Zustand vorliegt, schmaler ist als die maximale Haftmittelbreite (W71) durch das Haftmittel auf dem hydrophoben Vliesstoff (21, 31, 41) und wobei
das Taillenelement (20) Folgendes aufweist:
einen zweiten Hautseitenbereich (31B), der ein Bereich zwischen einem äußeren Ende des Haftmittels, das in dem hydrophoben Vliesstoff (21, 31, 41) bereitgestellt ist, und einem äußeren Ende des elastischen Taillenelements (35, 45) ist,
einen zweiten Nicht-Hautseitenbereich (32B), der ein Bereich ist, der dem zweiten Hautseitenbereich (31B) in dem hydrophilen Vliesstoff (22, 32, 42) zugewandt ist, und
einen ersten Nicht-Hautseitenbereich (32A), der ein Bereich ist, der eine vorgegebene Breite aufweist und an eine Außenseite des zweiten Nicht-Hautseitenbereichs (32B) in dem hydrophilen Vliesstoff (22, 32, 42) angrenzt, und
eine durchschnittliche Dichte des hydrophilen Vliesstoffs (22, 32, 42) in dem zweiten Nicht-Hautseitenbereich (32B) größer ist als eine durchschnittliche Dichte des hydrophilen Vliesstoffs (22, 32, 42) in dem ersten Nicht-Hautseitenbereich (32A).

2. Absorbierender Artikel (1, 2, 3) nach Anspruch 1, wobei
zumindest ein Teil des hydrophilen Vliesstoffs (22, 32, 42) am weitesten auf einer Nicht-Hautseite des Taillenelements (20) angeordnet ist.

3. Absorbierender Artikel (1, 2, 3) nach einem der Ansprüche 1 oder 2, wobei
das Taillenelement (20) einen vorderen Taillenteil (30) und einen hinteren Taillenteil (40) aufweist und
eine Schicht am weitesten auf einer Nicht-Hautseite von jedem des vorderen Taillenteils (30) und des hinteren Taillenteils (40) aus einer Lage von hydrophilem Vliesstoff ausgebildet ist.

4. Absorbierender Artikel (1) nach einem der Ansprüche 1 bis 3, wobei
der absorbierende Artikel (1) eine vertikale Richtung und eine laterale Richtung aufweist, die einander schneiden,
der absorbierende Hauptkörper (10) einen Saugkern (11) aufweist,
das Taillenelement (20) einen vorderen Taillenteil (30) und einen hinteren Taillenteil (40) aufweist,
der vordere Taillenteil (30) und der hintere Taillenteil (40) durch ein Paar von Seitenverbindungsteilen (50), die an zwei Endteilen in der lateralen Richtung bereitgestellt sind, ringförmig verbunden sind und,
hinsichtlich eines oberen Taillenbereichs (30U, 40U), der mit dem Seitenverbindungsteil (50) des Taillenelements (20) in Bezug auf die vertikale Richtung überlappt und der sich über einem oberen Ende des Saugkerns (11) in der vertikalen Richtung befindet,
hinsichtlich eines unteren Taillenbereichs (30D, 40D), der mit dem Seitenverbindungsteil (50) des Taillenelements (20) in Bezug auf die vertikale Richtung überlappt und der sich unter dem oberen Ende des Saugkerns (11) in der vertikalen Richtung befindet,
ein Verhältnis einer Gesamtfläche des Haftmittels, das in dem oberen Taillenbereich (30U, 40U) in einer linearen oder getüpfelten Weise angeordnet ist, zu einer Fläche des oberen Taillenbereichs (30U, 40U) größer ist als ein Verhältnis einer Gesamtfläche des Haftmittels, das in dem unteren Taillenbereich (30D, 40D) in einer linearen oder getüpfelten Weise angeordnet ist, zu einer Fläche des unteren Taillenbereichs (30D, 40D).

5. Absorbierender Artikel (1) nach Anspruch 4, wobei
das Taillenelement (20) eine Vielzahl von elastischen Taillenelementen (35, 45) aufweist, die sich in der lateralen Richtung dehnen und zusammenziehen, und
ein vertikaler Abstand zwischen der Vielzahl von elastischen Taillenelementen (35, 45), die in dem oberen Taillenbereich (30U, 40U) bereitgestellt sind, schmaler ist als ein vertikaler Abstand zwischen der Vielzahl von elastischen Taillenelementen (35, 45), die in dem unteren Taillenbereich (30D, 40D) bereitgestellt sind.

6. Absorbierender Artikel (1) nach Anspruch 4 oder 5, wobei
der hintere Taillenteil (40) eine Gesäßabdeckung (40b) unter unteren Enden der Seitenverbindungsteile (50) einschließt,
wobei die Gesäßabdeckung (40b) eine laterale Breite aufweist, die sich von einer oberen Seite zu einer unteren Seite in der vertikalen Richtung verengt,
der hintere Taillenteil (40) ein hinteres elastisches Beinelement (47) und ein elastisches Taillenelement (45) einschließt,
wobei sich das hintere elastische Beinelement (47) entlang eines Endrandteils der Gesäßabdeckung (40b) dehnt und zusammenzieht,
wobei sich das elastische Taillenelement (45) entlang der lateralen Richtung dehnt und zusammenzieht, und
das hintere elastische Beinelement (47) einen Teil aufweist, der das elastische Taillenelement (45) in dem Seitenverbindungsteil (50) schneidet.

7. Absorbierender Artikel (1) nach Anspruch 6, wobei
in einem äußeren Randteil (40be) der Gesäßabdeckung (40b) der absorbierende Artikel (1) einen Teil aufweist, in dem der hydrophile Vliesstoff (42) und der hydrophobe Vliesstoff (41) komprimiert sind.

8. Absorbierender Artikel (1) nach Anspruch 6 oder 7, wobei
in einem äußeren Randteil (40be) der Gesäßabdeckung (40b) der absorbierende Artikel (1) Folgendes aufweist:
einen Teil, in dem der hydrophile Vliesstoff (42) und der hydrophobe Vliesstoff (41) durch ein Haftmittel verbunden sind, und
einen Teil, in dem der hydrophile Vliesstoff (42) und der hydrophobe Vliesstoff (41) nicht verbunden sind.

9. Absorbierender Artikel (1) nach einem der Ansprüche 1 bis 8, wobei
der absorbierende Artikel (1) eine vertikale Richtung und eine laterale Richtung aufweist, die einander schneiden, und
eine Hautoberflächenlage (36, 46), die aus einem hydrophoben Vliesstoff ausgebildet ist, auf einer Hautseite des Taillenelements (20) bereitgestellt ist, um ein oberes Ende des absorbierenden Hauptkörpers (10) in der vertikalen Richtung zu überspannen.

10. Absorbierender Artikel (1) nach Anspruch 9, wobei
der absorbierende Artikel (1) einen Teil aufweist, in dem die Hautoberflächenlage (36, 46) und das Haftmittel, bei Ansicht in einer Dickenrichtung des Taillenelements (20), überlappen.

11. Absorbierender Artikel (1, 2, 3) nach Anspruch 9 oder 10, wobei
die Hautoberflächenlage (36, 46) beabstandet von einem oberen Ende des Taillenelements (20) um eine vorgegebene Entfernung in der vertikalen Richtung bereitgestellt ist.

## Revendications

1. Article absorbant (1, 2, 3), comportant :
un corps principal absorbant qui absorbe les liquides (10) ; et
un élément au niveau de la taille (20) qui est mis en œuvre sur un côté non orienté vers la peau par rapport au corps principal absorbant,
l'élément au niveau de la taille (20) ayant un tissu non tissé hydrophobe (21, 31, 41) et un tissu non tissé hydrophile (22, 32, 42) au moins dans une région partielle,
le tissu non tissé hydrophile (22, 32, 42)
qui est superposé de manière adjacente par rapport à un côté non orienté vers la peau du tissu non tissé hydrophobe (21, 31, 41) et
qui a une hydrophilie supérieure par rapport à celle du tissu non tissé hydrophobe (21, 31, 41),
l'élément au niveau de la taille (20) ayant une partie dans laquelle le tissu non tissé hydrophile (22, 32, 42) et le tissu non tissé hydrophobe (21, 31, 42) sont amenés à s'adhérer l'un par rapport à l'autre au moyen d'un adhésif,
une largeur adhésive maximale (W72) par l'adhésif dans le tissu non tissé hydrophile (22, 32, 42) est plus étroite qu'une largeur adhésive maximale (W71) par l'adhésif dans le tissu non tissé hydrophobe (21, 31, 41), dans lequel
l'article absorbant (1, 2, 3) a une direction verticale et une direction latérale qui se croisent l'une par rapport à l'autre,
l'élément au niveau de la taille (20) a un élément élastique au niveau de la taille (35, 45) qui s'étire et se contracte dans la direction latérale,
l'adhésif est mis en œuvre le long de l'élément élastique au niveau de la taille (35, 45), et
une largeur d'une section transversale de l'élément élastique au niveau de la taille (35, 45) qui est dans un état d'étirement dans la direction latérale est plus étroite que la largeur adhésive maximale (W71) par l'adhésif sur le tissu non tissé hydrophobe (21, 31, 41), et dans lequel
l'élément au niveau de la taille (20) a
une deuxième région côté orienté vers la peau (31B) qui est une région entre une extrémité extérieure de l'adhésif mis en œuvre dans le tissu non tissé hydrophobe (21, 31, 41) et une extrémité extérieure de l'élément élastique au niveau de la taille (35, 45),
une deuxième région côté non orienté vers la peau (32B) qui est une région faisant face à la deuxième région côté orienté vers la peau (31B) dans le tissu non tissé hydrophile (22, 32, 42), et
une première région côté non orienté vers la peau (32A) qui est une région ayant une largeur prédéterminée et adjacente par rapport à une partie extérieure de la deuxième région côté non orienté vers la peau (32B) dans le tissu non tissé hydrophile (22, 32, 42), et
une densité moyenne du tissu non tissé hydrophile (22, 32, 42) dans la deuxième région côté non orienté vers la peau (32B) est supérieure à une densité moyenne du tissu non tissé hydrophile (22, 32, 42) dans la première région côté non orienté vers la peau (32A).

2. Article absorbant (1, 2, 3) selon la revendication 1, dans lequel
au moins une partie du tissu non tissé hydrophile (22, 32, 42) est agencée au plus loin sur un côté non orienté vers la peau de l'élément au niveau de la taille (20).

3. Article absorbant (1, 2, 3) selon l'une quelconque des revendications 1 ou 2, dans lequel
l'élément au niveau de la taille (20) a une partie avant au niveau de la taille (30) et une partie arrière au niveau de la taille (40), et
une couche située le plus loin sur le côté non orienté vers la peau de chacune parmi la partie avant au niveau de la taille (30) et la partie arrière au niveau de la taille (40) est formée à partir d'une feuille de tissu non tissé hydrophile.

4. Article absorbant (1) selon l'une quelconque des revendications 1 à 3, dans lequel
l'article absorbant (1) a une direction verticale et une direction latérale qui se croisent l'une par rapport à l'autre,
le corps principal absorbant (10) a une partie centrale absorbante (11),
l'élément au niveau de la taille (20) a une partie avant au niveau de la taille (30) et une partie arrière au niveau de la taille (40),
la partie avant au niveau de la taille (30) et la partie arrière au niveau de la taille (40) sont assemblées de manière annulaire par une paire de parties d'assemblage latérales (50) qui sont mises en œuvre au niveau de deux parties d'extrémité dans la direction latérale, et
en ce qui concerne une région supérieure au niveau de la taille (30U, 40U) qui chevauche la partie d'assemblage latérale (50) de l'élément au niveau de la taille (20) par rapport à la direction verticale et qui est située au-dessus d'une extrémité supérieure de la partie centrale absorbante (11) dans la direction verticale,
en ce qui concerne une région inférieure au niveau de la taille (30D, 40D) qui chevauche la partie d'assemblage latérale (50) de l'élément au niveau de la taille (20) par rapport à la direction verticale et qui est située sous l'extrémité supérieure de la partie centrale absorbante (11) dans la direction verticale,
un rapport entre une surface totale de l'adhésif se trouvant dans la région supérieure au niveau de la taille (30U, 40U) de manière linéaire ou pointillée et une surface de la région supérieure au niveau de la taille (30U, 40U) est supérieur à un rapport entre une surface totale de l'adhésif se trouvant dans la région inférieure au niveau de la taille (30D, 40D) de manière linéaire ou pointillée et une surface de la région inférieure au niveau de la taille (30D, 40D).

5. Article absorbant (1) selon la revendication 4, dans lequel
l'élément au niveau de la taille (20) a une pluralité d'éléments élastiques au niveau de la taille (35, 45) qui s'étirent et se contractent dans la direction latérale, et
un pas vertical entre les éléments de la pluralité d'éléments élastiques au niveau de la taille (35, 45) qui sont mis en œuvre dans la région supérieure au niveau de la taille (30U, 40U) est plus étroit qu'un pas vertical entre les éléments de la pluralité d'éléments élastiques au niveau de la taille (35, 45) qui sont mis en œuvre dans la région inférieure au niveau de la taille (30D, 40D).

6. Article absorbant (1) selon la revendication 4 ou la revendication 5, dans lequel
la partie arrière au niveau de la taille (40) comprend un couvre-fesses (40b) sous les extrémités inférieures des parties d'assemblage latérales (50),
le couvre-fesses (40b) ayant une largeur latérale qui va en se rétrécissant depuis un côté supérieur jusqu'à un côté inférieur dans la direction verticale,
la partie arrière au niveau de la taille (40) comprend un élément élastique de jambe arrière (47) et un élément élastique au niveau de la taille (45),
l'élément élastique de jambe arrière (47) s'étirant et se contractant le long d'une partie formant bord d'extrémité du couvre-fesses (40b),
l'élément élastique au niveau de la taille (45) s'étirant et se contractant le long de la direction latérale, et
l'élément élastique de jambe arrière (47) a une partie qui croise l'élément élastique au niveau de la taille (45), dans la partie d'assemblage latérale (50).

7. Article absorbant (1) selon la revendication 6, dans lequel
dans une partie formant bord extérieur (40be) du couvre-fesses (40b), l'article absorbant (1) a une partie dans laquelle le tissu non tissé hydrophile (42) et le tissu non tissé hydrophobe (41) sont comprimés.

8. Article absorbant (1) selon la revendication 6 ou la revendication 7, dans lequel
dans une partie formant bord extérieur (40be) du couvre-fesses (40b), l'article absorbant (1) a
une partie dans laquelle le tissu non tissé hydrophile (42) et le tissu non tissé hydrophobe (41) sont assemblés par un adhésif et
une partie dans laquelle le tissu non tissé hydrophile (42) et le tissu non tissé hydrophobe (41) ne sont pas assemblés.

9. Article absorbant (1) selon l'une quelconque des revendications 1 à 8, dans lequel
l'article absorbant (1) a une direction verticale et une direction latérale qui se croisent l'une par rapport à l'autre, et
une feuille de surface orientée vers la peau (36, 46) formée à partir d'un tissu non tissé hydrophobe est mise en œuvre sur un côté orienté vers la peau de l'élément au niveau de la taille (20), de manière à chevaucher une extrémité supérieure du corps principal absorbant (10) dans la direction verticale.

10. Article absorbant (1) selon la revendication 9, dans lequel
l'article absorbant (1) a une partie dans laquelle la feuille de surface orientée vers la peau (36, 46) et l'adhésif se chevauchent quand ils sont vus dans la direction allant dans le sens de l'épaisseur de l'élément au niveau de la taille (20).

11. Article absorbant (1, 2, 3) selon la revendication 9 ou la revendication 10, dans lequel
la feuille de surface orientée vers la peau (36, 46) est mise en œuvre de manière espacée par rapport à une extrémité supérieure de l'élément au niveau de la taille (20) d'une distance prédéterminée dans la direction verticale.
